(19) Europäisches Patentamt
European Patent Office
Office européen des brevets

(11) **EP 3 042 413 B1**

(12) **EUROPÄISCHE PATENTSCHRIFT**

(45) Veröffentlichungstag und Bekanntmachung des Hinweises auf die Patenterteilung:
**25.10.2017  Patentblatt 2017/43**

(21) Anmeldenummer: **14789127.9**

(22) Anmeldetag: **19.08.2014**

(51) Int Cl.:
*H01M 8/0206* (2016.01)    *H01M 8/0247* (2016.01)
*H01M 8/0258* (2016.01)    *B22F 9/20* (2006.01)
*C22C 1/08* (2006.01)    *C22C 1/10* (2006.01)
*C22C 27/06* (2006.01)    *C22C 32/00* (2006.01)
*C22C 1/04* (2006.01)    *B22F 5/00* (2006.01)
*H01M 8/124* (2016.01)    *B22F 3/24* (2006.01)

(86) Internationale Anmeldenummer:
**PCT/AT2014/000158**

(87) Internationale Veröffentlichungsnummer:
**WO 2015/027254 (05.03.2015 Gazette 2015/09)**

(54) **PULVERMETALLURGISCHES FORMTEIL ALS INTERKONNEKTOR ODER ENDPLATTE FÜR EINE ELEKTROCHEMISCHE ZELLE**

BODY FROM METALLURGIC POWDER AS INTERCONNECTOR OR ENDPLATTE FOR AN ELECTROCHEMICAL CELL

FORME À PARTIR DE POUDRE MÉTALLURGIQUE COMME INTERCONNECTEUR OU PLAQUE TERMINALE POUR CELLULE ÉLECTROCHIMIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **02.09.2013  AT 2822013 U**

(43) Veröffentlichungstag der Anmeldung:
**13.07.2016  Patentblatt 2016/28**

(73) Patentinhaber: **Plansee SE**
**6600 Reutte (AT)**

(72) Erfinder:
• **BRANDNER, Marco**
**87448 Waltenhofen (DE)**
• **O'SULLIVAN, Michael**
**A-6600 Ehenbichl (AT)**

• **LEITER, Thomas**
**A-6600 Reutte (AT)**
• **HIRSCH, Oliver**
**A-6622 Berwang (AT)**
• **KRAUSSLER, Wolfgang**
**A-6671 Weißenbach (AT)**

(74) Vertreter: **Ciesla, Bettina**
**Plansee Group Service GmbH**
**Intellectual Property Department**
**6600 Reutte (AT)**

(56) Entgegenhaltungen:
**EP-A1- 0 378 812        EP-A1- 0 629 015**
**EP-A1- 2 230 707        WO-A1-97/35349**
**WO-A1-2013/010198**

**Beschreibung**

Die vorliegende Erfindung betrifft ein einen Interkonnektor oder eine Endplatte für eine elektrochemische Zelle bildendes, pulvermetallurgisches Formteil, das einen Chromgehalt von mindestens 80 Gew.%, eine plattenförmige Grundform und (ein) an einer oder an beiden Hauptflächen des Formkörpers ausgebildete(s) Strömungsfeld(er) aufweist, wobei die jeweilige Hauptfläche in dem Bereich des/der Strömungsfeldes/Strömungsfelder jeweils eine in dem Formkörper ausgebildete Strukturierung mit einer Mehrzahl von noppen- oder stegförmigen Erhebungen und dazwischen liegenden Vertiefungen aufweist. Die vorliegende Erfindung betrifft ferner ein Herstellungsverfahren für solch ein pulvermetallurgisches Formteil.

Festoxidbrennstoffzellen (solid oxide fuel cells - SOFCs) sowie weitere, ähnlich aufgebaute, elektrochemische Zellen, wie beispielsweise Festoxidelektrolysezellen (solid oxide electrolyzer cells - SOECs), sind für relativ hohe Betriebstemperaturen (z.B. bei elektrolytgestützten, elektrochemischen Zellen typischerweise im Bereich zwischen 700 °C und 950 °C) ausgelegt. Unter anderem sind elektrochemische Zellen (SOFCs, SOECs) bekannt, die eine keramische Festelektrolytplatte (z.B. auf Basis von Zirkonoxid mit geringen Zusätzen von Yttriumoxid oder Scandiumoxid) mit beidseitig ausgebildeten Elektroden (Anode, Kathode) aufweisen. Häufig werden die elektrochemischen Zellen als ebene Einzelelemente ausgebildet und zu einem Stapel (engl.: stack) übereinander gestapelt. Innerhalb des Stapels ist die Führung der Prozessgase zu den einzelnen elektrochemischen Zellen, die Trennung der Prozessgase benachbarter, elektrochemischer Zellen sowie die elektrische Kontaktierung zwischen jeweils benachbarten, elektrochemischen Zellen zu bewerkstelligen. Zur Bereitstellung dieser Funktionen werden zwischen den einzelnen, elektrochemischen Zellen eines Stapels jeweils Interkonnektoren eingesetzt und der Stapel selbst wird in der Regel durch Endplatten, die die genannten Funktionen nur zu einer Seite hin bereitstellen und ggf. zur anderen Seite hin entsprechende Anschlussoptionen aufweisen, abgeschlossen. Bei Brennstoffzellenanwendungen umfasst die Führung der Prozessgase beispielsweise die anodenseitige Brenngaszuführung, die kathodenseitige Sauerstoffzuführung (z.B. Luft) sowie die anodenseitige und kathodenseitige Ableitung der entstandenen Gase.

Interkonnektoren und Endplatten müssen weiterhin einen an die elektrochemische Zelle (insbesondere an den Festelektrolyten) angepassten, thermischen Ausdehnungskoeffizienten sowie eine hohe Korrosionsbeständigkeit aufweisen. Ausgehend von diesen Anforderungen haben sich Cr-Basislegierungen (d.h. Cr-Gehalt ≥ 50 Gew.%, insbesondere Cr-Gehalt ≥ 80 Gew.%, vgl. z.B. US 5,407,758 A) bewährt. Bei der Herstellung von Interkonnektoren und Endplatten ist die pulvermetallurgische Fertigungsroute, die zumindest die Schritte des Pressens von einem entsprechenden Pulveransatz und des Sinterns aufweist, etabliert. Sie zeichnet sich u.a. durch vergleichsweise niedrige Herstellungskosten aus.

Je nach Anwendungsgebiet bilden die volumetrische und ggf. auch die gravimetrische Leistungsdichte (d.h. die Leistung pro Volumen bzw. die Leistung pro Gewicht) des elektrochemischen Systems, welches eine Mehrzahl von elektrochemischen Zellen und dazwischen angeordnete Interkonnektoren (sowie ggf. endseitig vorgesehene Endplatten) umfasst, ein wesentliches Kriterium. Pulvermetallurgisch hergestellte Interkonnektoren gemäß ihrer bisher bekannten Bauformen sind relativ dick ausgebildet und nehmen z.B. bei elektrolytgestützten, elektrochemischen Zellen ca. 15 mal so viel Bauraum wie die einzelnen, elektrochemischen Zellen in Anspruch. Die relativ dicke Ausbildung von herkömmlichen, pulvermetallurgischen Interkonnektoren und Endplatten ist u.a. bedingt durch die nachfolgenden Faktoren:

a) Bereitstellung von an einer oder an beiden Hauptfläche(n) ausgebildeten Strömungsfeld(ern) mit Strukturierung(en);
b) Bereitstellung einer ausreichenden Stabilität des Formteils;
c) Erzielung einer gleichmäßigen Temperaturverteilung über das Formteil; und
d) Bereitstellung einer prozesssicheren Fertigungsroute.

Dementsprechend besteht die Aufgabe der vorliegenden Erfindung darin, Interkonnektoren und Endplatten im Hinblick auf die Erzielung einer möglichst hohen volumetrischen und gravimetrischen Leistungsdichte des zugehörigen elektrochemischen Systems weiter zu optimieren, wobei gleichzeitig niedrige Bauteilkosten für die Interkonnektoren und Endplatten angestrebt werden.

Die Aufgabe wird durch ein pulvermetallurgisches Formteil gemäß Anspruch 1 sowie durch ein Verfahren zum Herstellen von solch einem Formteil gemäß Anspruch 15 gelöst. Vorteilhafte Weiterbildungen der Erfindung sind in den abhängigen Ansprüchen angegeben.

Gemäß der vorliegenden Erfindung wird ein pulvermetallurgisches Formteil bereitgestellt, das einen Interkonnektor oder eine Endplatte für eine elektrochemische Zelle bildet. Das Formteil weist einen Chromgehalt von mindestens 80 Gew.%, eine plattenförmige Grundform und (ein) an einer oder an beiden Hauptflächen des Formkörpers ausgebildete(s) Strömungsfeld(er) auf. Die jeweilige Hauptfläche weist in dem Bereich des/der Strömungsfeldes/Strömungsfelder jeweils eine in dem Formkörper ausgebildete Strukturierung mit einer Mehrzahl von noppen- oder stegförmigen Erhebungen und dazwischen liegenden Vertiefungen auf. Ein Verhältnis aus dem maximalen, entlang der Hauptfläche ge-

messenem Durchmesser $D_{max}$ (in mm) des Formteils relativ zu einer minimalen Dicke $d_{min}$ (in mm) eines sich entlang des/der Strömungsfeldes/Strömungsfelder erstreckenden und von der/den Strukturierung(en) nicht betroffenen Kernbereiches des Formteils liegt in dem Bereich $140 \leq D_{max}/d_{min} \leq 350$.

Innerhalb dieses Bereiches sind die Formteile im Vergleich zu bisher bekannten, pulvermetallurgischen Interkonnektoren und Endplatten relativ zu deren maximalem Durchmesser dünner ausgebildet. Dadurch kann für das zugehörige, elektrochemische System (bestehend aus einer Mehrzahl von elektrochemischen Zellen und Interkonnektoren sowie ggf. endseitig vorgesehenen Endplatten) eine höhere volumetrische sowie gravimetrische Leistungsdichte erzielt werden. Ein weiterer Vorteil ist, dass die für das einzelne Formteil anfallenden Materialkosten reduziert sind. Durch eine solche relative (d.h. im Verhältnis zu dem maximalen Durchmesser $D_{max}$) Reduzierung der Dicke des Kernbereichs kann eine relativ hohe Strukturtiefe der Strukturierung(en) des/der Strömungsfeldes/Strömungsfelder beibehalten werden. Dadurch wird ein verhältnismäßig großer Strömungsquerschnitt für die Prozessgase bereitgestellt, was wiederum für die Prozessgasführung vorteilhaft ist (da weniger Druckverlust über die Kanallänge hinweg auftritt). Vorzugsweise beträgt die Strukturtiefe $\geq 0,5$ mm, wobei vorzugsweise die Strukturtiefe auf der der Kathode zugewandten Hauptfläche tiefer (z.B. $\geq 0,8$ mm) als die Strukturtiefe auf der der Anode zugewandten Hauptfläche (z.B. $\geq 0,5$ mm) ist. Die beanspruchte Bereichsangabe für das Verhältnis $D_{max}/d_{min}$ ist deshalb eine sehr gut geeignete Größe zur Definition der Größenverhältnisse des Formteils, da sie eine von der äußeren Formgebung und von der absoluten Gesamtgröße des Formteils unabhängige Kenngröße ist und da sich gezeigt hat, dass aus Stabilitätsgründen des Formteils (vgl. Kriterium b) oberhalb) und aufgrund der fertigungstechnischen Rahmenbedingungen (vgl. Kriterium d) oberhalb) bei der pulvermetallurgischen Herstellung die Dicke des Kernbereiches mit zunehmender Größe des Formteils zunehmen sollte. Der maximale Wert des beanspruchten Bereichs für das Verhältnis $D_{max}/d_{min}$ ist derart gewählt, dass noch eine ausreichende Stabilität gegeben ist und dass über das Formteil hinweg eine weitgehend gleichmäßige Temperaturverteilung gewährleistet wird (vgl. Kriterien b) und c) oberhalb).

In Bezug auf eine möglichst hohe Stabilität der Formteile ist anzumerken, dass bisher für die pulvermetallurgische Fertigung von Interkonnektoren und Endplatten in der Regel Chrompulver bzw. Chrom enthaltende Pulver eingesetzt werden, die über einen vergleichsweise kostengünstigen, aluminothermischen Prozess hergestellt werden. Aluminothermische Verfahren gehen von fein verteiltem Aluminiumpulver aus, welches sorgfältig mit Chromoxid vermischt wird. Anschließend wird diese Reaktionsmischung entzündet, wodurch das Chromoxid zu metallischem Chrom reduziert wird, während das metallische Aluminium zu $Al_2O_3$ oxidiert wird. Das entstandene Reaktionsprodukt wird geschmolzen, wobei sich flüssiges Chrom absetzt und Schlacke mit dem Hauptbestandteil $Al_2O_3$ auf dem flüssigen Chrom schwimmt. Nach Abkühlung und Erstarrung wird die Schlacke vom reinen Chrom-Metall mechanisch abgetrennt, das Metall wird in Klumpen zerbrochen und zur Gewinnung des Chrommetallpulvers auf die gewünschte Teilchengröße bzw. Teilchengrößenverteilung vermahlen, wobei sich ggf. auch noch Siebvorgänge anschließen können. Das so erhaltene Pulver, das herkömmlich für die Formteil-Fertigung eingesetzt wird, ist - sofern keine zusätzlichen, oberflächenvergrößernden Bearbeitungsschritte durchgeführt werden - grob und kantig ausgebildet und weist nahezu keine innere Porosität auf. Die Verwendung solch eines Pulvers (ggf. in Kombination mit weiteren Pulvern, Presshilfsmitteln, etc.) ermöglicht zwar verhältnismäßig hohe Pressdichten. Es hat sich jedoch gezeigt, dass die Stabilität des gepressten Grünlings wie auch des gesinterten Formteiles bei Verwendung solcher Pulver vergleichsweise niedrig ist. Dies wird auf eine nur mäßige Verklammerung zwischen den einzelnen Partikeln sowie auf eine relativ ungleichmäßige Verteilung der Restporosität zurückgeführt. In den gesinterten Formteilen, die aus solchen Pulvern hergestellt sind, liegen einzelne, verhältnismäßig große Poren vor. Dementsprechend können die erfindungsgemäß definierten Formteile mit dem oberhalb beschriebenen Pulver in der Regel nicht mit einer ausreichenden Stabilität hergestellt werden.

Es hat sich gezeigt, dass wenn bei dem pulvermetallurgischen Herstellungsvorgang des Formteils zumindest ein Anteil des Chrom enthaltenen Pulvers eine Oberfläche nach BET von $\geq 0,05$ m²/g aufweist, eine deutlich höhere Stabilität des gepressten Grünlings wie auch des gesinterten Formteils erzielt werden kann. Die Angaben zur Oberfläche nach BET im Rahmen dieser Anmeldung beziehen sich auf eine BET-Messung gemäß Norm (ISO 9277:1995, Messbereich: 0,01 - 300 m²/g; Gerät: Gemini II 2370, Ausheiztemperatur: 130°C, Ausheizzeit: 2 Stunden; Adsorptiv: Stickstoff, volumetrische Auswertung über Fünfpunktbestimmung). Die Verwendung solch eines Pulvers ermöglicht die Herstellung der erfindungsgemäß definierten Formteile mit einer besonders hohen Stabilität. Dementsprechend betrifft die vorliegende Erfindung auch ein pulvermetallurgisches Formteil gemäß Anspruch 1, das gemäß dem in Anspruch 15 definierten Verfahren herstellbar ist, insbesondere das gemäß dem in Anspruch 15 definierten Verfahren hergestellt ist. Wie in Bezug auf Weiterbildungen und Varianten der vorliegenden Erfindung beschrieben wird, kann solch ein Chrom enthaltendes Pulver durch oberflächenvergrößernde Bearbeitungsschritte von herkömmlichen Pulvern (z.B. aluminothermisch hergestellt) oder auch durch ein Herstellungsverfahren (vgl. Anspruch 19), bei welchem zumindest eine Verbindung der Gruppe bestehend aus Chromoxid und Chromhydroxid, optional mit einer beigemischten festen Kohlenstoffquelle, unter zumindest zeitweiser Einwirkung von Wasserstoff und Kohlenwasserstoff reduziert wird, hergestellt werden. Das so hergestellte Pulver weist eine schwammartige Struktur auf und lässt sich besonders gut und dicht pressen (selbst bei niedrigeren Pressdrücken), wobei eine gute Verzahnung zwischen den Partikeln erreicht wird. Dadurch lässt sich das Formteil nach Erreichen des Maximaldrucks ohne Auftreten von Materialausbrüchen wieder entlasten. Ferner wird eine hohe Grünfestigkeit der Grünlinge und eine hohe Stabilität der gesinterten Formteile erzielt.

Als pulvermetallurgisches Formteil wird ein in der beschriebenen Formgebung (d.h. einschließlich der Strukturierung(en)) durch Pressen von Pulver(n) und durch nachfolgendes Sintern hergestelltes Bauteil bezeichnet, wobei auch weitere Behandlungsschritte, wie beispielsweise ein Vorsintern nach dem Schritt des Pressens und ggf. auch ein Kalibrierpressen nach dem Schritt des Vorsinterns (und vor dem Schritt des Sinterns), vorgesehen sein können. Das Formteil in seiner beschriebenen Formgebung (d.h. einschließlich der Strukturierung(en)) ist dabei monolithisch ausgebildet und weist durchgehend die im Rahmen der pulvermetallurgischen Herstellung erhaltene Mikrostruktur auf. Insbesondere wird die beschriebene Formgebung ausschließlich durch entsprechend ausgebildete Presswerkzeuge erzielt und es erfolgt kein nachfolgender, formgebender Bearbeitungsschritt. Die im Rahmen der pulvermetallurgischen Herstellung erhaltene Mikrostruktur entsteht dadurch, dass die durch Pressen verdichteten Partikel mit Fortschreiten des Sinterprozesses durch Festkörperdiffusion miteinander über Sinterhälse verbunden werden, die zunehmend dicker werden, während die Zwischenräume zwischen den Partikeln zunehmend kleiner werden. Es liegt eine statistische Verteilung der Größe und der Position der Poren vor. Ein "pulvermetallurgisches Formteil" ist für einen Fachmann durch metallurgische Untersuchungsmethoden (z.B. Bildung eines Schliffes an einer Querschnittsfläche und Untersuchung im Rasterelektronenmikroskop) erkennbar. Das pulvermetallurgische Formteil kann mit weiteren Anbauteilen, Dichtungsabschnitten und/oder Beschichtungen, etc., versehen sein.

Die Zusammensetzung des Formteils liegt insbesondere innerhalb des nachfolgenden Bereichs:

$$\text{Cr-Gehalt (Cr: Chrom) von} \geq 88 \text{ Gew.\% und} \leq 98 \text{ Gew.\%}$$

$$\text{Fe-Gehalt (Fe: Eisen) von} \geq 2 \text{ Gew.\% und} \leq 12 \text{ Gew.\%}$$

gegebenenfalls Zusätze, z.B. Y-Zusatz (Y: Yttrium) (z.B. 0,07 Gew.% Y).

Auf diese Weise kann eine hohe Korrosionsbeständigkeit und eine gute Anpassung des thermischen Ausdehnungskoeffizienten an das Material des Elektrolyten, der vorzugsweise aus (z.B. mit $Y_2O_3$ oder $Sc_2O_3$) vollstabilisiertem Zirkonoxid gebildet wird (insbesondere bei elektrolytgestützten, elektrochemischen Zellen), erreicht werden. Gemäß einer Weiterbildung beträgt der Chromanteil (bezogen auf den Gesamtmetallgehalt) $\geq 90$ Gew.%. Mit zunehmendem Chromgehalt wird die Wärmeleitfähigkeit erhöht (homogene Temperaturverteilung erreichbar) und der thermische Ausdehnungskoeffizient abgesenkt (bessere Anpassung desselben an verfügbare Elektrolytmaterialien, wie beispielsweise vollstabilisiertes Zirkonoxid). Vorzugsweise wird das Formteil durch einen Interkonnektor, der an beiden Hauptflächen jeweils mindestens ein Strömungsfeld aufweist, gebildet. Die Strukturierung(en) können dabei beispielsweise durch ein Rillenfeld mit zwischen den einzelnen Rillen angeordneten, länglichen Erhebungen (in Form von z.B. Stegen, Rippen, etc.), die ggf. auch entlang ihrer Haupterstreckungsrichtung ein- oder mehrmalig unterbrochen sein können, gebildet werden. Daneben können - wie dem Fachmann geläufig ist - für die Prozessgasführung auch andere Arten von Strukturierungen geeignet sein.

Das erfindungsgemäße Formteil (Interkonnektor oder Endplatte) ist für verschiedene Arten von Festoxid-basierten elektrochemischen Zellen geeignet. Vorzugsweise ist es für elektrolytgestützte, elektrochemische Zellen, insbesondere Brennstoffzellen, ausgebildet. Bei der elektrolytgestützten Zelle bildet der Elektrolyt die tragende Komponente für die angrenzenden Elektroden (und ggf. weitere Schichten). Gemäß einer Weiterbildung ist das Formteil für eine elektrolytgestützte, elektrochemische Zelle (insbesondere Brennstoffzelle) mit (z.B. mit $Y_2O_3$ oder $Sc_2O_3$) vollstabilisiertem Zirkonoxid als Elektrolyt ausgebildet. Solch ein Elektrolyt zeichnet sich durch eine hohe Ionenleitfähigkeit und damit eine hohe Leistungsfähigkeit aus, er ist aber auch sehr bruchempfindlich, so dass eine besonders gute Anpassung an dessen thermischen Ausdehnungskoeffizienten erforderlich ist. Hierfür ist das beanspruchte Formteil mit dem vergleichsweise hohen Chromgehalt (sowie auch mit dem bevorzugten Zusammensetzungsbereich) besonders gut geeignet. Für die Form der Hauptfläche sind grundsätzlich unterschiedliche Geometrien möglich, wie beispielsweise rechteckig, quadratisch, rautenförmig, Parallelogramm, kreisförmig, oval, etc., ggf. auch mit einer oder mehreren Öffnung(en) innerhalb der Hauptfläche. Die Hauptfläche ist eben oder im Wesentlichen eben (d.h. sie weist nur presstechnisch bedingte Neigungen oder Welligkeiten auf) ausgebildet. Der Durchmesser wird bezogen auf die einfassenden Kanten des Formteils gemessen, unabhängig davon, ob eine oder mehrere Öffnung(en) innerhalb der Hauptfläche ausgebildet sind. Auch kann/können auf einer Hauptfläche nicht nur exakt ein Strömungsfeld sondern auch mehrere, voneinander unterteilte Strömungsfelder vorgesehen sein. Die Dicke wird senkrecht zu der Erstreckungsebene der Hauptfläche(n) gemessen. Gemäß der vorliegenden Erfindung verbleibt in Richtung der Dicke in dem Bereich des/der Strömungsfeldes/Strömungsfelder ein nicht von den Strukturierung(en) betroffener Kernbereich, d.h. ein Bereich, den die von einer oder von beiden Hauptflächen in den Formkörper ragenden Vertiefungen nicht erreichen. Die Dicke dieses Kernbereiches in dem Bereich des/der Strömungsfeldes/Strömungsfelder kann entlang der Ebene der Hauptfläche variieren und es wird dementsprechend auf die minimale Dicke Bezug genommen. Ggf. kann eine minimale Dicke des Formteils an Abschnitten außerhalb des/der Strömungsfelder auch dünner oder dicker sein.

Im Hinblick auf eine weitergehende Reduzierung der Materialkosten und eine Optimierung der volumetrischen und gravimetrischen Leistungsdichte des zugehörigen, elektrochemischen Systems ist bevorzugt, wenn das Verhältnis $D_{max}/d_{min} \geq 150$, insbesondere $\geq 180$ ist. Im Hinblick auf eine gleichmäßige Temperaturverteilung und eine ausreichende Stabilität ist umgekehrt vorteilhaft, wenn das Verhältnis $D_{max}/d_{min} \leq 300$, insbesondere $\leq 250$, ist.

Gemäß einer Weiterbildung der vorliegenden Erfindung ist an beiden Hauptflächen des Formteils genau ein Strömungsfeld ausgebildet. Insbesondere ist der Kernbereich zwischen den beiden, einander gegenüberliegenden Strukturierungen der Strömungsfelder ausgebildet. Gemäß einer Weiterbildung ist ein das Strömungsfeld vollständig oder teilweise umgebender (und von Strukturierung(en) des jeweils angrenzenden Strömungsfeldes freier) Randbereich an der jeweiligen Hauptfläche vorgesehen. In dem Randbereich können zusätzlich eine oder mehrere (z.B. schlitzförmige) Durchgangsöffnung(en), die der Zu- oder Abführung der Prozessgase dienen, vorgesehen sein. Die Führung der Prozessgase kann zumindest teilweise auch außerhalb der Erstreckungsfläche der Interkonnektoren (entlang z.B. entsprechender, außenseitig verlaufender Kanäle) erfolgen und die Strukturierungen erstrecken sich in diesem Fall bis an die jeweilige Außenkante des Interkonnektors. Es können auch beide Varianten an einem Interkonnektor, z.B. an verschiedenen Seitenkanten, vorgesehen sein.

Gemäß einer Weiterbildung ist/sind an beiden Hauptflächen des Formteils jeweils (ein) Strömungsfeld(er) ausgebildet und die Strukturierungen voneinander gegenüberliegenden Strömungsfeldern weisen im Wesentlichen parallel zueinander verlaufende Haupterstreckungsrichtungen (z.B. der Stege, der Rippen, der länglich ausgebildeten Noppen, der Kanäle, der Rillen, etc., der jeweiligen Strukturierung) entlang der Hauptflächen auf (Gleichstromdesign). Mit "im Wesentlichen parallel" wird auf einen exakt parallelen Verlauf Bezug genommen, sowie alternativ auch auf einen nahezu parallelen Verlauf mit Abweichungen von bis zu $\pm$ 10°. Das Gleichstromdesign hat unter anderem den Vorteil, dass bei dem Pressvorgang des Formteils ein bevorzugter Materialfluss und eine gleichmäßige Materialverteilung erzielbar ist. Insbesondere ist in dieser Hinsicht bevorzugt, wenn die Vertiefungen der einen Hauptfläche exakt oder im Wesentlichen gegenüberliegend von den Erhebungen der anderen Hauptfläche angeordnet sind und umgekehrt. Weiterhin ist das erfindungsgemäße Formteil auch als Interkonnektor im Kreuzstromdesign ausführbar, bei welchem die Haupterstreckungsrichtung der Strukturierung des einen Strömungsfeldes senkrecht oder im Wesentlichen senkrecht (d.h. Abweichungen von bis zu $\pm$ 10°) relativ zu der Haupterstreckungsrichtung der Strukturierung eines gegenüberliegend angeordneten Strömungsfeldes ausgerichtet ist. Daneben sind auch anderweitige, z.B. schräg zueinander verlaufende Orientierungen oder auch über die Hauptfläche zueinander wechselnde Orientierungen von einander gegenüberliegenden Strömungsfeldern möglich. Weiterhin ist im Hinblick auf den Pressvorgang des Formteils vorteilhaft, wenn die Flanken der Strukturierung(en) jeweils derart geneigt sind, dass sich die Vertiefungen zu deren Grund hin jeweils beidseitig verjüngen (und sich entsprechend die Erhebungen zu deren höchsten Punkt hin beidseitig verjüngen).

Gemäß einer Weiterbildung weist das Formteil vier umlaufende Seitenkanten auf ("umlaufend" um die jeweilige Hauptfläche) von denen jeweils zwei einander gegenüberliegende Seitenkanten parallel zueinander verlaufen, wobei das Verhältnis aus der Seitenlänge $L_{max}$ der längsten Seitenkante relativ zu der minimalen Dicke $d_{min}$ des Kernbereiches des Formteils $\geq 110$ ist. Die beschriebene Geometrie des Formteils ist insbesondere für den Aufbau eines Stapels und für die Prozessgasführung (an den vier umlaufenden Seiten) vorteilhaft. Insbesondere weist das Formteil eine rechteckige, gegebenenfalls auch quadratische Grundform auf und es liegt das Verhältnis gemäß dieser Weiterbildung vor. Im Hinblick auf eine weitergehende Reduzierung der Materialkosten und eine Optimierung der Leistungsdichte ist bevorzugt, wenn das Verhältnis $\geq 115$, insbesondere $\geq 150$ ist. Im Hinblick auf eine gleichmäßige Temperaturverteilung und eine ausreichende Stabilität ist umgekehrt vorteilhaft, wenn das Verhältnis $\leq 220$ ist. Ferner ist im Hinblick auf die Gesamtleistung eines Stacks bevorzugt, wenn sämtliche Seitenlängen $\geq 155$ mm, insbesondere $\geq 170$ mm sind. Gemäß einer Weiterbildung ist das Verhältnis aus der Größe der Hauptfläche in mm² (Quadratmillimeter) relativ zu der minimalen Dicke $d_{min}$ des Kernbereiches des Formteils in mm (Millimeter) $\geq 1,3 \times 10^4$. Dieses Verhältnis ist unabhängig von der Geometrie der Hauptfläche eine geeignete Größe zur Beschreibung bevorzugter geometrischer Verhältnisse des Formteils, wobei ggf. in der Hauptfläche vorgesehene Öffnungen (z.B. für die Prozessgasführung) mit zu der Größe der Hauptfläche gezählt werden. Im Hinblick auf die Materialkosten und die Gesamtleistung des Stacks ist bevorzugt, wenn das Verhältnis $\geq 1,5 \times 10^4$, insbesondere $\geq 2,0 \times 10^4$ ist. Im Hinblick auf eine gleichmäßige Temperaturverteilung und eine ausreichende Stabilität ist umgekehrt vorteilhaft, wenn das Verhältnis $\leq 3,5 \times 10^4$ ist. Gemäß einer Weiterbildung ist die Größe der Hauptfläche $\geq 240$ cm² (Quadratzentimeter), vorzugsweise $\geq 280$ cm², wobei die Größe der Hauptfläche vorzugsweise $\leq 500$ cm² (Quadratzentimeter) ist.

Gemäß einer Weiterbildung ist das Verhältnis aus Gesamtgewicht des Formteils in g (Gramm) relativ zu der Größe der Hauptfläche in cm² (Quadratzentimeter) $\leq 1,1$ ist. Dementsprechend wird eine hohe gravimetrische Leistungsdichte und ein niedriger Materialeinsatz erzielbar. Insbesondere ist unter diesen Gesichtspunkten vorteilhaft, wenn das Verhältnis $\leq 1,0$, insbesondere $\leq 0,9$ ist, wobei aus Stabilitätsgründen bevorzugt ist, wenn das Verhältnis $\geq 0,7$ ist.

Gemäß einer Weiterbildung ist die maximale Dicke (gemessen senkrecht zu der Erstreckungsebene der Hauptfläche(n)) des Formteils in dem Bereich des/der Strömungsfeldes/Strömungsfelder $\leq 2,3$ mm (Millimeter), insbesondere ≤ 2,1 mm. Um einen ausreichenden Strömungsquerschnitt für die Prozessgase bereitzustellen, ist die maximale Dicke vorzugsweise ≥ 1,6 mm. Vorzugsweise gelten diese Grenzwerte auch für die maximale Dicke des gesamten Formteils.

Gemäß einer Weiterbildung ist die minimale Dicke $d_{min}$ des Kernbereiches des Formteils ≤ 1,1 mm (Millimeter), insbesondere ≤ 1,0 mm, gegebenenfalls sogar ≤ 0,9 mm. Im Hinblick auf eine ausreichende Stabilität ist bevorzugt, wenn die minimale Dicke ≥ 0,7 mm beträgt. Gemäß einer Weiterbildung ist der maximale, entlang der Hauptfläche gemessene Durchmesser des Formteils ≥ 200 mm. Im Hinblick auf eine möglichst hohe Gesamtleistung des Stacks ist der maximale, entlang der Hauptfläche gemessene Durchmesser des Formteils ≥ 220 mm, insbesondere ≥ 250 mm, wobei aus Stabilitätsgründen vorzugsweise ein Wert ≤ 320 mm gewählt wird.

Gemäß einer Weiterbildung liegt in dem Kernbereich der Flächenanteil P von leeren oder teilweise mit Metalloxid(en) gefüllten Poren sowie von Oxideinschlüssen, soweit sie vollständig innerhalb der betrachteten Messfläche liegen, relativ zu der Gesamtfläche dieser Messfläche in einem Bereich von 80 % ≤ (1-P) ≤ 95 %, (zu Messverfahren s. oben). P würde bei einem Material, in dem keine Metalloxide aus Cr oder weiteren Metallen gebildet sind, der Porosität (ausgewertet mittels quantitativer Bildanalyse) entsprechen, wobei gemäß der vorliegenden Erfindung die (ursprünglichen) Poren teils oder vollständig mit Metalloxid(en) aufgefüllt sein können. Die mit Metalloxid(en) gefüllten Poren sowie die Oxideinschlüsse können zumindest zum Teil gezielt eingebracht werden, indem das Formteil nach Durchlaufen der pulvermetallurgischen Herstellungsroute einem anschließenden Oxidationsprozess unterzogen wird, um die offene Restporosität zu minimieren. Denn das entstehende $Cr_2O_3$ oder Mischoxide aus Cr und Al (siehe US 2010/0233576 A1) weisen ein größeres Volumen als die metallische Matrix auf, so dass die Porosität im Zuge des Oxidationsprozesses verschlossen werden kann. Dabei werden nicht notwendigerweise alle Poren bis zum Kern des Formteils mit Oxiden gefüllt, zumindest wird jedoch eine Randschicht von ca. 0,2 mm Dicke geschlossen. Die entstehende Oxidschicht auf der Oberfläche des Formteils wird insbesondere in einem nachfolgenden Prozess zumindest in dem Bereich der elektrischen Kontaktflächen wieder entfernt, beispielsweise durch einen Sandstrahlprozess, um zu Betriebsbeginn einen möglichst optimalen, metallischen Kontakt zwischen elektrochemischer Zelle und Interkonnektor bzw. Endplatte zu gewährleisten. Dementsprechend werden bei der Beschreibung der Mikrostruktur des Formteils in den meisten Fällen Poren, teilweise mit Metalloxid(en) gefüllte Poren sowie Oxideinschlüsse zusammengefasst. Bei teilweise mit Metalloxid(en) gefüllten Poren wird deren Querschnittsfläche (im Rahmen der quantitativen Bildanalyse) berechnet, indem der teilweise gefüllte Anteil und der leere Anteil zusammenaddiert werden. Der Hauptbestandteil der "Oxideinschlüsse" sowie auch der "Metalloxid(e)" im Rahmen der Erfindung sind Chromoxide, wobei der Chromoxidgehalt bevorzugt mindestens 90 Gew.% beträgt. Die "Oxideinschlüsse" und "Metalloxid(e)" können aber auch noch Metalloxide weiterer, vorhandener Metalle sowie auch Metallnitride von Chrom und/oder weiteren, vorhandenen Metallen enthalten.

Weist bei dem pulvermetallurgischen Herstellungsvorgang des Formteils zumindest ein Anteil des Chrom enthaltenden Pulvers eine Oberfläche nach BET von ≥ 0,05 m²/g (Quadratmeter/Gramm) auf, so kann - wie oberhalb erläutert wird - eine deutlich höhere Stabilität erzielt werden und das Pulver lässt sich sehr gut pressen. Insbesondere ist der Anteil des Chrom enthaltenden Pulvers mit einer Oberfläche nach BET von ≥ 0,05 m²/g ≥ 5 Gew.%, vorzugsweise ≥ 10 Gew.%, jeweils bezogen auf die Gesamtpulvermenge der Metall-enthaltenden Pulver (ggf. elementar und/oder als Oxid, Nitrid, Karbid, etc. vorliegend) des Pulveransatzes für das Formteil (Wachs als Presshilfsmittel wird diesbezüglich z.B. nicht mit eingerechnet). Vorzugsweise wird der gesamte Chrom-Anteil des Pulveransatzes für das Formteil durch solch ein Chrom enthaltendes Pulver mit einer Oberfläche nach BET von ≥ 0,05 m²/g bereitgestellt. Insbesondere sind für die Herstellung des Formteils die Verfahrensschritte gemäß Anspruch 15 anzuwenden. Die Verwendung solch eines Pulvers ist auch an der vorteilhaften Mikrostruktur des Formteils erkennbar, deren bevorzugte Merkmale nachfolgend unter Bezugnahme auf Weiterbildungen erläutert werden. Die Mikrostruktur zeichnet sich durch fein verteilte, sehr kleine Poren aus. Dies ermöglicht im Vergleich zu herkömmlich hergestellten Formteilen, die Bauteilstärke (insbesondere im Kernbereich) zu reduzieren und/oder die Formteile deutlich größer (bezogen auf deren Hauptfläche) auszubilden. Auch ergibt sich dadurch die Möglichkeit, das Formteil gasdicht mit deutlich reduziertem Anteil an Porenfüller (z.B. durch gezielte Oxidation eingebrachte Metalloxide, insbesondere Chromoxid) oder ggf. auch unter Verzicht auf den Oxidationsprozess zu fertigen, was einerseits zu besser kontrollierbaren, physikalischen Eigenschaften führt und andererseits die Fertigungskosten senkt. Ein Pulver mit einer Oberfläche nach BET von ≥ 0,05 m²/g kann zum Einen bereitgestellt werden, indem oberflächenvergrößernde Bearbeitungsschritte an herkömmlich (z.B. über einen aluminothermischen Prozess) hergestellten Chrompulvern oder Chrom-basierten Pulvern vorgenommen werden. Beispielsweise kann eine Oberflächenvergrößerung erzielt werden, indem solch ein herkömmliches Pulver sehr fein vermahlen wird, danach agglomeriert wird (z.B. in einen Mischer oder über eine Sprühagglomeration), nachfolgend einem Glühprozess (z.B. in einem Temperaturbereich von 800 - 1.200 °C und über eine Dauer von ca. 1 h (Stunde)) unterzogen wird und dann gesiebt wird. Besonders gute Eigenschaften des Formteils, die anhand von dessen Mikrostruktur erkennbar sind, werden erzielt, wenn das Pulver mit einer Oberfläche nach BET von ≥ 0,05 m²/g gemäß den in Anspruch 19 angegebenen Verfahrensschritten hergestellt wird. Durch solch ein Verfahren ist (ohne Durchführung von oberflächenvergrößernden Bearbeitungsvorgängen) insbesondere ein Pulver herstellbar, das einen BET-Wert von ≥ 0,25 m²/g, insbesondere von bis zu 0,5 m²/g, aufweist. Das so hergestellte Pulver weist eine schwammartige Struktur (der einzelnen Partikel) auf und lässt sich dadurch besonders gut pressen. Insbesondere sind niedrigere Pressdrücke im Vergleich zu herkömmlichem Pulver möglich.

Gemäß einer Weiterbildung ist in dem Kernbereich der Flächenanteil von leeren oder teilweise mit Metalloxid(en) gefüllten Poren sowie von Oxideinschlüssen, die jeweils eine Fläche von $\geq$ 100 $\mu m^2$ (Quadratmikrometer) aufweisen, relativ zu der Gesamtfläche an leeren oder teilweise mit Metalloxid(en) gefüllten Poren und Oxideinschlüssen $\leq$ 60 %. Diese Flächen werden dabei mittels quantitativer Bildanalyse an einer rasterelektronenmikroskopischen Aufnahme einer Messfläche, die in eine entlang der Dickenrichtung verlaufende Schnittfläche durch das Formteil in den Kernbereich gelegt wird, ausgewertet, wobei das Messverfahren unterhalb im Detail erläutert wird (vgl. "Beschreibung der quantitativen Bildanalyse zur Bestimmung der Mikrostruktur"). Insbesondere liegt der Flächenanteil $\leq$ 40%, vorzugsweise $\leq$ 10%, und noch bevorzugter $\leq$ 7%, wobei die niedrigeren Werte in Bezug auf die Stabilität bevorzugt sind und insbesondere dann erreicht werden (z.B. wurde ein Flächenanteil von 5% erreicht), wenn ein gemäß Anspruch 19 hergestelltes Pulver zumindest anteilig eingesetzt wird, insbesondere wenn der Cr-Anteil vollständig durch solch ein gemäß Anspruch 19 hergestelltes Pulver bereitgestellt wird.

Gemäß einer Weiterbildung ist in dem Kernbereich der Flächenanteil von leeren oder teilweise mit Metalloxid(en) gefüllten Poren sowie von Oxideinschlüssen, die jeweils eine Fläche von $\geq$ 70 $\mu m^2$ (Quadratmikrometer) aufweisen, relativ zu der Gesamtfläche an leeren oder teilweise mit Metalloxid(en) gefüllten Poren und Oxideinschlüssen $\leq$ 70 % (zu Messverfahren s. oben). Insbesondere liegt der Flächenanteil $\leq$ 50%, vorzugsweise $\leq$ 20%, und noch bevorzugter $\leq$ 17%, wobei die niedrigeren Werte in Bezug auf die Stabilität bevorzugt sind und insbesondere dann erreicht werden (z.B. wurde ein Flächenanteil von 14% erreicht), wenn ein gemäß Anspruch 19 hergestelltes Pulver zumindest anteilig eingesetzt wird, insbesondere wenn der Cr-Anteil vollständig durch solch ein gemäß Anspruch 19 hergestelltes Pulver bereitgestellt wird.

Gemäß einer Weiterbildung ist in dem Kernbereich der Flächenanteil von leeren oder teilweise mit Metalloxid(en) gefüllten Poren sowie von Oxideinschlüssen, die jeweils eine Fläche von $\geq$ 50 $\mu m^2$ (Quadratmikrometer) aufweisen, relativ zu der Gesamtfläche an leeren oder teilweise mit Metalloxid(en) gefüllten Poren und Oxideinschlüssen $\leq$ 80 % (zu Messverfahren s. oben). Insbesondere liegt der Flächenanteil $\leq$ 60%, vorzugsweise $\leq$ 30%, und noch bevorzugter $\leq$ 27%, wobei die niedrigeren Werte in Bezug auf die Stabilität bevorzugt sind und insbesondere dann erreicht werden (z.B. wurde ein Flächenanteil von 24% erreicht), wenn ein gemäß Anspruch 19 hergestelltes Pulver zumindest anteilig eingesetzt wird, insbesondere wenn der Cr-Anteil vollständig durch solch ein gemäß Anspruch 19 hergestelltes Pulver bereitgestellt wird.

Gemäß einer Weiterbildung liegt in dem Kernbereich eine gleichmäßige Verteilung der leeren oder teilweise mit Metalloxid(en) gefüllten Poren sowie der Oxideinschlüsse vor (wobei das Verhältnis zwischen leeren Poren, teilweise gefüllten Poren und Oxideinschlüssen lokal variieren kann; z.B. kann - wie oberhalb erläutert wird - der Anteil an Metalloxid-Füllung und an Oxideinschlüssen relativ zu leerem Porenvolumen zu der Oberfläche des Formteils hin zunehmen) und der mittlere Abstand $\lambda$ der leeren oder teilweise mit Metalloxid(en) gefüllten Poren und der Oxideinschlüsse ist $\leq$ 9 $\mu m$, wobei der mittlere Abstand $\lambda$ nach der Formel

$$\lambda = \left( \frac{\frac{4}{3}\pi a^3}{P} \right)^{\frac{1}{3}}$$

berechnet wird.

Dabei entspricht 2a dem mittleren, äquivalenten Poren- bzw. Oxideinschlussdurchmesser (ermittelt gemäß der Formel $2a = \frac{\sum_{i=1}^{n}(2a_i)}{n}$, wobei "$2a_i$" jeweils die äquivalenten Poren- bzw. Oxideinschlussdurchmesser der vollständig innerhalb der betrachteten Messfläche liegenden leeren oder teilweise mit Metalloxid(en) gefüllten Poren und Oxideinschlüsse sind). Ferner ist P der Flächenanteil von leeren oder teilweise mit Metalloxid(en) gefüllten Poren sowie von Oxideinschlüssen, soweit sie vollständig innerhalb der betrachteten Messfläche liegen (zu dem Messverfahren für die Ermittlung von 2a und P mittels quantitativer Bildanalyse siehe oben). Der äquivalente Poren- bzw. Oxideinschlussdurchmesser $2a_i$ wird bei Poren und Oxideinschlüssen unabhängig von der konkreten Querschnittsform, die nicht zwingend kreisförmig ist, aus der Querschnittsfläche A derselben gemäß der Formel $2a_i = (4A/\pi)^{0,5}$ ermittelt. Insbesondere ist der mittlere Abstand $\lambda \leq$ 7 $\mu m$, vorzugsweise $\leq$ 5 $\mu m$, wobei die niedrigeren Werte in Bezug auf die Stabilität bevorzugt sind und insbesondere dann erreicht werden (erreicht wurde z.B. $\lambda$ = 4 $\mu m$, wobei $\lambda$ typischerweise $\geq$ 2 $\mu m$ ist), wenn ein gemäß Anspruch 19 hergestelltes Pulver zumindest anteilig eingesetzt wird, insbesondere wenn der Cr-Anteil vollständig durch solch ein gemäß Anspruch 19 hergestelltes Pulver bereitgestellt wird. Bei herkömmlichen, aluminothermischen Pulver liegt typischerweise ein mittlerer Abstand $\lambda$ von 12,7 $\mu m$ vor.

Die oberhalb erläuterten Merkmale, welche die Mikrostruktur des Kernbereichs betreffen, liegen vorzugsweise durchgehend in dem gesamten Kernbereich des jeweiligen Formteils vor. Vorzugsweise liegen sie durchgehend in dem gesamten Formteil vor.

Die vorliegende Erfindung betrifft ferner ein Verfahren zum Herstellen eines pulvermetallurgischen, erfindungsgemäßen Formteils, das gegebenenfalls gemäß einer oder mehrerer der oberhalb erläuterten Weiterbildungen und Varianten ausgebildet sein kann.

Das Verfahren weist dabei nachfolgende Schritte auf:

A) Bereitstellen eines Pulveransatzes, der bezogen auf den Gesamtmetallgehalt einen Chromanteil von $\geq$ 80 Gew.% enthält und bei dem zumindest ein Anteil des Chrom enthaltenden Pulvers eine Oberfläche nach BET von $\geq$ 0,05 $m^2/g$, insbesondere $\geq$ 0,07 $m^2/g$, vorzugsweise $\geq$ 0,25 $m^2/g$, aufweist;

B) Pressen des Pulveransatzes zu einem Pressling (insbesondere bei einem Pressdruck im Bereich von 500 bis 1.000 MPa);

C) Sintern des Presslings bei 1.100 bis 1.500 °C (vorzugsweise unter Wasserstoffatmosphäre).

Vorzugsweise wird der gesamte Cr-Anteil des Formteils durch ein gemäß Anspruch 19 hergestelltes Pulver bereitgestellt. Aus Kostengründen kann es aber auch vorteilhaft sein, Pulver mit einer Oberfläche nach BET von $\geq$ 0,05 $m^2/g$, das insbesondere gemäß dem Verfahren nach Anspruch 19 hergestellt ist, mit herkömmlichem Pulver (z.B. aluminothermisch hergestellt) zu mischen, wodurch ebenfalls noch eine ausreichende Stabilität und eine gute Pressbarkeit erzielt werden können.

Gemäß einer Weiterbildung der Erfindung erfolgt zwischen dem Schritt des Pressens (Schritt B) und dem Schritt des Sinterns (Schritt C) ein Vorsintern des Presslings bei 500 bis 1.000 °C (vorzugsweise unter Wasserstoffatmosphäre). Gemäß einer Weiterbildung wird dem Pulveransatz vor dem Verpressen ein Presshilfsmittel (z.B. ein Wachs) in einer Menge von 0,1 Gew.% bis 5 Gew.%, bezogen auf die Pulveransatzmenge, zugesetzt.

Gemäß einer Weiterbildung wird das nach dem Schritt des Sinterns (Schritt C) erhaltene Formteil unter Anwesenheit einer Sauerstoffquelle (z.B. $O_2$, $H_2O$, $CO_2$, oder Mischungen daraus; ggf. auch weitere Sauerstoffquellen möglich) oxidiert (bei einer Temperatur im Bereich von 800 - 1.050 °C) und anschließend wird die Oxidschicht von zumindest einem Teil der Oberfläche des Formteils entfernt.

Gemäß einer Weiterbildung bildet der Schritt des Pressens (Schritt B)) den einzigen, während der Herstellung des Formteils durchgeführten Pressvorgang. Bei herkömmlichen Pulvern ist in der Regel ein zweimaliges Pressen erforderlich, um die geforderte Präzision der Konturen zu erreichen. Durch die Verwendung von Chrom-enthaltenden Pulvern, die eine Oberfläche nach BET von $\geq$ 0,05 $m^2/g$ aufweisen, insbesondere von Chrom-enthaltenden Pulvern, die gemäß Anspruch 19 hergestellt wurden, ist bereits in einem einmaligen Pressvorgang und bei vergleichsweise niedrigen Pressdrücken eine präzise Ausbildung der Konturen erzielbar. Zudem vorteilhaft sind vergleichsweise niedrige Herstellungskosten.

Alternativ zu der einstufigen Herstellung (d.h. mit nur einem Pressschritt) können im Rahmen der pulvermetallurgischen Herstellung auch zwei Pressschritte vorgesehen werden. In letzterem Fall wird insbesondere zwischen dem Schritt des Vorsinterns und dem Schritt des Sinterns (Schritt C)) ein Kalibrierpressvorgang bei einem Pressdruck im Bereich von 500 bis 1.000 MPa durchgeführt. Dadurch wird insbesondere eine Homogenisierung der Dichteverhältnisse im Formteil erzielt.

Gemäß einer Weiterbildung wird das oberhalb erwähnte Verfahren gemäß Anspruch 19 zur Herstellung eines Chrom enthaltenden Pulvers angewendet und dem Pulveransatz zumindest anteilig zugegeben. Das erfindungsgemäße Metallpulver lässt sich durch Reduktion zumindest einer Verbindung der Gruppe bestehend aus Chromoxid und Chromhydroxid, optional mit einer beigemischten festen Kohlenstoffquelle, unter zumindest zeitweiser Einwirkung von Wasserstoff und Kohlenwasserstoff herstellen.

Als Chromoxid oder Chromhydroxid kommen bevorzugt Cr(III)- Verbindungen in Pulverform in Frage, beispielsweise $Cr_2O_3$, CrOOH, $Cr(OH)_3$ oder natürlich Mischungen aus Chromoxiden und Chromhydroxiden. Die bevorzugte Chromquelle ist $Cr_2O_3$ (vorzugsweise in Pigmentqualität).

Bevorzugt wird die Verbindung der Gruppe bestehend aus Chromoxid und Chromhydroxid, optional mit einer beigemischten festen Kohlenstoffquelle, auf eine Temperatur $T_R$ mit 1100°C $\leq$ $T_R$ $\leq$ 1550°C, vorzugsweise auf eine Temperatur $T_R$ mit 1200°C $\leq$ $T_R$ $\leq$ 1450°C, erhitzt und optional auf dieser Temperatur gehalten. Während im unteren Temperaturbereich sehr lange Haltezeiten auf $T_R$ erforderlich sind, um einen vorteilhaften Reduktionsgrad von 90% einzustellen, kann im oberen Temperaturbereich die Haltezeit sehr kurz gewählt werden oder überhaupt entfallen. Der Gesamtdruck der Reaktion beträgt in vorteilhafter Weise 0,95 bis 2 bar. In vorteilhafter Weise liegt der Kohlenwasserstoff als $CH_4$ vor. Bevorzugt beträgt zumindest während des Aufheizvorgangs zumindest zeitweise der Kohlenwasserstoff-Partialdruck 5 bis 500 mbar. Die Restgasatmosphäre ist dabei bevorzugt Wasserstoff.

Bevorzugt erfolgt die Einwirkung von Wasserstoff und Kohlenwasserstoff zumindest im Temperaturbereich 800°C bis 1050°C. Bevorzugt liegt in diesem Temperaturbereich der Kohlenwasserstoff-Partialdruck in einem Bereich von 5 bis 500 mbar. Die sich aus den Ausgangsstoffen bildende Reaktionsmischung befindet dabei bevorzugt zumindest 45 min., insbesondere bevorzugt zumindest 60 min., in diesem Temperaturbereich. Diese Zeit schließt sowohl den Aufheizvorgang als auch etwaige isotherme Haltephasen in diesem Temperaturbereich ein. Mit den erfinderischen Verfahrensbedingungen ist gewährleistet, dass sich bei Temperaturen bevorzugt $< T_R$ der Kohlenwasserstoff mit zumindest einer Verbindung ausgewählt aus der Gruppe bestehend aus Chromoxid und Chromhydroxid in Anwesenheit von Wasserstoff teilweise zu Chromkarbid umsetzt. Bevorzugte Chromkarbide sind $Cr_3C_2$, $Cr_7C_3$ und $Cr_{23}C_6$. Die sich über den Kohlenwasserstoff-Partialdruck einstellende zumindest teilweise Bildung von Chromkarbid wirkt sich günstig auf die Pulvereigenschaften aus. Mit den beschriebenen Verfahrensbedingungen ist ferner gewährleistet, dass sich das Chromkarbid mit dem in der Reaktionsmischung vorhandenen oder neu hinzugefügten Chromoxid und/oder Chromhydroxid zu Chrom (Cr) umsetzt, wobei dieser Prozess bei $T_R$ dominiert.

Der Kohlenwasserstoff kann der Reaktion gasförmig, bevorzugt ohne Zumischen einer festen Kohlenstoffquelle, zugegeben werden. Bevorzugt wird dabei die Verbindung der Gruppe bestehend aus Chromoxid und Chromhydroxid unter zumindest zeitweiser Einwirkung eines $H_2$-$CH_4$ Gasgemisches reduziert. Vorteilhaft wird ein $H_2/CH_4$ Volumenverhältnis im Bereich 1 bis 200, besonders vorteilhaft von 1,5 bis 20 gewählt. Die Einwirkung des $H_2$-$CH_4$ Gasgemisches erfolgt dabei bevorzugt zumindest zeitweise während der Aufheizphase auf $T_R$, wobei der Einfluss auf die Ausbildung der Pulverform insbesondere im Temperaturbereich 850 bis 1000°C sehr günstig ist. Wird eine Temperatur von ca. 1200°C erreicht, wird bevorzugt auf eine Rein-Wasserstoffatmosphäre, bevorzugt mit einem Taupunkt von < -40°C (gemessen im Bereich der Gaszufuhr) umgeschaltet. Liegt $T_R$ unter 1200°C, erfolgt das Umschalten auf die Rein-Wasserstoffatmosphäre bevorzugt bei Erreichen von $T_R$. Die isotherme Phase auf $T_R$ und Abkühlen auf Raumtemperatur erfolgen vorteilhaft in einer Wasserstoffatmoshäre. Insbesondere beim Abkühlen ist es vorteilhaft, Wasserstoff mit einem Taupunkt < -40°C zu verwenden, um Rückoxidation zu vermeiden.

In einer Ausführungsvariante wird dem Chromoxid und/oder Chromhydroxid eine feste Kohlenstoffquelle zugemischt. Bevorzugt wird dabei pro Mol Sauerstoff in der Chromverbindung zwischen 0,75 und 1,25 Mol, vorzugsweise zwischen 0,90 und 1,05 Mol an Kohlenstoff eingesetzt. Dabei ist die Menge an für die Reaktion mit der Chromverbindung verfügbaren Kohlenstoff gemeint. In einer besonders bevorzugten Ausführungsvariante ist das Verhältnis O zu C mit etwa 0,98 leicht unterstöchiometrisch. Bevorzugt ist vorgesehen, dass die feste Kohlenstoffquelle ausgewählt ist aus der Gruppe Ruß, Aktivkohle, Graphit, kohlenstofffreisetzende Verbindungen oder Mischungen daraus. Als Beispiel für eine kohlenstofffreisetzende Verbindung können Chromkarbide, wie zum Beispiel $Cr_3C_2$, $Cr_7C_3$ und $Cr_{23}C_6$ genannt werden. Die Pulvermischung wird in einer $H_2$-haltigen Atmosphäre auf $T_R$ erhitzt. Der $H_2$-Druck wird dabei bevorzugt so eingestellt, dass sich zumindest im Temperaturbereich 800° bis 1050°C ein $CH_4$-Partialdruck von 5 bis 500 mbar ergibt. Die isotherme Phase auf $T_R$ und Abkühlen auf Raumtemperatur erfolgen wiederum vorteilhaft in einer Wasserstoffatmoshäre. Während dieser Prozessphase ist die Anwesenheit von Kohlenwasserstoff nicht erforderlich. Wasserstoff verhindert während dieser Prozessphase und während der Abkühlphase Rückoxidationsprozesse. Während der Abkühlphase wird bevorzugt eine Wasserstoffatmosphäre mit einem Taupunkt < -40°C eingesetzt.

Weitere Vorteile und Zweckmäßigkeiten der Erfindung ergeben sich anhand der nachfolgenden Beschreibung von Ausführungsbeispielen unter Bezugnahme auf die beigefügten Figuren.

Von den Figuren zeigen:

Fig. 1: eine Draufsicht eines Interkonnektors im Gleichstromdesign;
Fig. 2: eine Querschnittsansicht eines Ausschnitts des in Fig. 1 gezeigten Interkonnektors;
Fig. 3: eine Querschnittsansicht eines Ausschnitts einer Endplatte;
Fig. 4: eine rasterelektronenmikroskopische Aufnahme eines Interkonnektors mit vorteilhafter Mikrostruktur; und
Fig. 5: eine rasterelektronenmikroskopische Aufnahme eines Interkonnektors mit herkömmlicher Mikrostruktur.

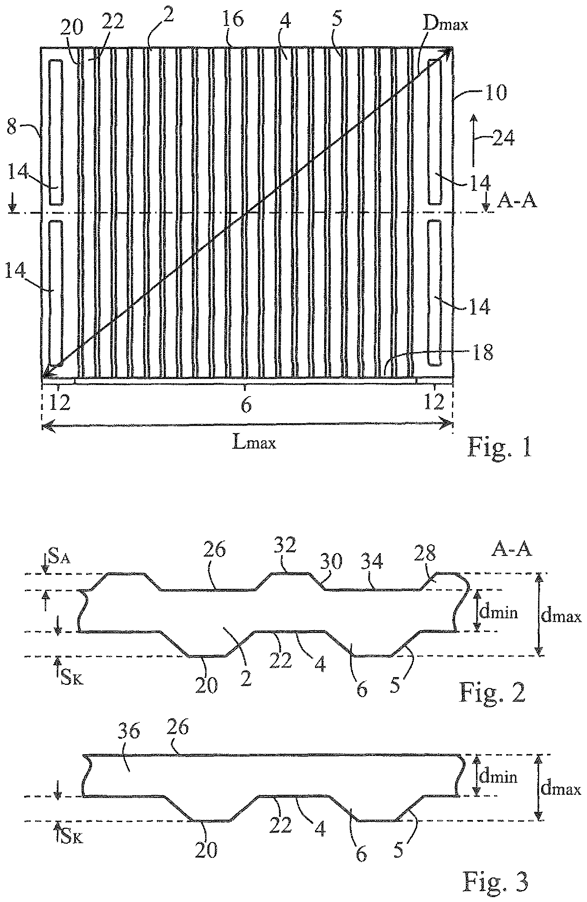

In Fig. 1 ist eine Draufsicht auf eine Hauptfläche 4 eines (rechteckig ausgebildeten) Interkonnektors 2 im Gleichstromdesign gezeigt. Die Hauptfläche 4 wird durch vier umlaufende Seitenkanten 8, 10, 16, 18 begrenzt. Der Interkonnektor 2 weist an der gezeigten Hauptfläche 4 genau ein Strömungsfeld 6 mit einer in dem Interkonnektor 2 ausgebildeten Strukturierung 5 auf. An das Strömungsfeld 6 grenzt an zwei, einander gegenüberliegenden Seiten jeweils ein Randbereich 12 an, der sich bis zu den Seitenkanten 8 bzw. 10 erstreckt. In den Randbereichen 12 sind jeweils schlitzförmige Durchgangsöffnungen 14 für die Zu- und Abführung der Prozessgase vorgesehen. An den verbleibenden zwei Seiten erstreckt sich das Strömungsfeld 6 bis zu den jeweiligen Seitenkanten 16, 18. Das Strömungsfeld 6 weist eine Mehrzahl von stegförmigen Erhebungen 20 mit dazwischen liegenden Vertiefungen 22 auf, die sich jeweils im Wesentlichen parallel zueinander entlang einer Haupterstreckungsrichtung 24 durchgehend von der einen Seitenkante 16 bis zu der gegenüberliegenden Seitenkante 18 des Interkonnektors 2 erstrecken (in Fig. 1 nur schematisch angedeutet). Die Flanken der stegförmigen Erhebungen 20 fallen jeweils schräg in Richtung zu den Vertiefungen 22 ab (vgl. Fig. 2). In Fig. 2 ist ein Ausschnitt des in Fig. 1 gezeigten Interkonnektors 2 im Bereich des Strömungsfeldes 6 in Querschnittsansicht (entlang der in Fig. 1 dargestellten Schnittfläche A-A) dargestellt. Auf der gegenüberliegenden Hauptfläche 26 ist ebenfalls genau ein Strömungsfeld 28 mit einer Strukturierung 30, die eine Mehrzahl von stegförmigen Erhebungen 32 mit dazwischen liegenden Vertiefungen 34 aufweist, in entsprechender Weise ausgebildet. Angrenzend an die Seitenkanten 8, 10 sind wiederum (nicht dargestellte) Randbereiche vorgesehen. Dabei verlaufen die Haupterstreckungsrichtungen 24 der Strukturierungen 5, 30 beider Hauptflächen 4, 26 parallel zueinander (Gleichstromdesign), wobei die Vertiefungen 22 der einen Hauptfläche 4 jeweils gegenüberliegend zu den Erhebungen 32 der anderen Hauptfläche 26 angeordnet sind und umgekehrt (Anordnung auf Lücke).

Wie anhand der Fig. 2 ersichtlich ist, ist vorliegend die Strukturtiefe $S_A$ der gegenüberliegenden Hauptfläche 26, die im Einsatz z.B. einer Anode einer angrenzenden Brennstoffzelle zugewandt ist, niedriger als die Strukturtiefe $S_K$ der in Fig. 1 dargestellten Hauptfläche 4, die im Einsatz z.B. einer Kathode einer angrenzenden Brennstoffzelle zugewandt ist. Der maximale, entlang der Hauptfläche 4 bzw. 26 gemessene Durchmesser $D_{max}$ entspricht bei der dargestellten Formgebung des Interkonnektors 2 dem Abstand zweier gegenüberliegender Ecken, wie in Fig. 1 dargestellt ist. Die Seitenlänge der längsten Seitenkante $L_{max}$ ist in Fig. 1 ebenfalls dargestellt. Die Dicke des von den Strukturierungen 5, 30 nicht betroffenen Kernbereiches im Bereich der Strömungsfelder 6, 28 ist bei dem dargestellten Interkonnektor 2 im Wesentlichen konstant über die Hauptflächen 4, 26 hinweg und entspricht damit der minimalen Dicke $d_{min}$ (vgl. Fig. 2). Die maximale Dicke $d_{max}$ des Interkonnektors 2 entspricht bei der dargestellten Ausführungsform Abstand der Erhebungen 20, 32 im Bereich der Strömungsfelder 6, 28, projiziert auf eine senkrecht zu der Ebene der Hauptflächen 4, 26 verlaufenden Dickenrichtung, und ist über den Bereich der Strömungsfelder 6, 28 hinweg konstant (vgl. Fig. 2).

In Fig. 3 ist eine Querschnittsansicht eines Ausschnitts einer Endplatte 36 dargestellt. Diese weist nur an einer Hauptfläche 4, die (bei der dargestellten Ausführungsform) im Einsatz der Kathode einer angrenzenden Brennstoffzelle zugewandt ist, ein Strömungsfeld 6 mit einer Strukturierung 5 in entsprechender Weise, wie dies in Bezug auf die Fig. 1 und 2 erläutert wurde, auf. Dementsprechend werden in Fig. 3 für einander entsprechende Bauteilabschnitte die gleichen Bezugszeichen wie in den Fig. 1 und 2 verwendet und es wird auf die Erläuterungen der Fig. 1 und 2 verwiesen. Im Unterschied zu den Fig. 1 und 2 grenzt im Bereich des Strömungsfeldes 6 an den Kernbereich nur zu einer Seite hin eine Strukturierung 5 an, so dass die minimale Dicke $d_{min}$ des Kernbereiches sowie die maximale Dicke $d_{max}$ der Endplatte 36 jeweils von den Vertiefungen 22 bzw. Erhebungen 20 bis zu der gegenüberliegenden Hauptfläche 26 entlang der Dickenrichtung gemessen wird (vgl. Fig. 3).

Nachfolgend werden Ausführungsbeispiele zur Herstellung von Interkonnektoren gemäß der vorliegenden Erfindung erläutert. Es wurden dabei Interkonnektoren mit unterschiedlichen Geometrien hergestellt, die alle ein Verhältnis $D_{max}/d_{min}$ im Bereich von $140 \leq D_{max}/d_{min} \leq 350$ aufwiesen und gleichzeitig eine ausreichende Stabilität zeigten. Insbesondere wurden Interkonnektoren mit den nachfolgend angegebenen Abmessungen hergestellt.

| Nr.: | Länge [mm] | Breite [mm] | $d_{max}$ [mm] | $d_{min}$ [mm] | $D_{max}/d_{min}$ |
|---|---|---|---|---|---|
| 1 | 100 | 100 | 1,8 | 0,89 | 158,9 |
| 2 | 110 | 110 | 1,8 | 0,9 | 172,8 |
| 3 | 180 | 180 | 2,5 | 1,0 | 254,6 |
| 4 | 200 | 160 | 2,5 | 1,0 | 256,1 |
| 5 | Hauptfläche rund: Durchmesser: 200 mm | | 2,5 | 1,0 | 200,0 |

Chrompulvergewinnung mit einer großen Oberfläche nach BET:

500 g $Cr_2O_3$ in Pigmentqualität (Lanxess Bayoxide CGN-R) mit einer mittleren, mittels Laserbeugung gemessenen Teilchengröße $d_{50}$ von 0,9 $\mu$m (Pulvermorphologie siehe Figur 3) wurde in $H_2$(75vol.%)-$CH_4$(25vol.%) (Durchflussrate 150 l/h, Druck ca. 1bar) in 80 min. auf 800°C erhitzt. In weiterer Folge wurde die Reaktionsmischung langsam auf 1200°C erhitzt, wobei sich die Reaktionsmischung 325 min. im Temperaturbereich 800 bis 1200°C befand. Danach wurde die Reaktionsmischung in 20 min. auf $T_R$ erhitzt mit $T_R$= 1400°C. Die Haltezeit auf 1400°C betrug 180 min. Aufheizen von 1200°C auf $T_R$ und Halten auf $T_R$ erfolgten unter Zufuhr von trockenem Wasserstoff mit einem Taupunkt < -40°C, wobei der Druck ca. 1 bar betrug. Die Ofenabkühlung erfolgte ebenfalls unter $H_2$ mit einem Taupunkt < -40°C. Es wurde ein metallischer Schwamm erhalten, der sehr leicht zu einem Pulver deagglomeriert werden konnte.

Pulveransatz:

Anschließend wird ein Pulveransatz, bestehend aus 95 Gew.-% feinem Cr-Pulver (mit einer Oberfläche nach BET von $\geq$ 0,05 m$^2$/g, granuliert zu einem besser rieselfähigen Pulver mit einer Partikelgrößenfraktion von 45 - 250 $\mu$m, z. B. hergestellt nach dem oberhalb erläuterten Verfahren zur Chrompulvergewinnung) und 5 Gew.-% einer FeY-Vorlegierung (Legierung mit 0,8 Gew.% Y, Partikelgröße < 100 $\mu$m) hergestellt. Dem Pulveransatz wird 1 Gew.-% Presshilfsmittel (Wachs) zugesetzt. Anschließend wird diese Mischung über 15 min in einem Taumelmischer vermischt.

Ausführungsbeispiel Einfaches Pressen:

Diese Mischung wird in eine Matrize gefüllt und bei einem spezifischen Pressdruck von 500 bis 1000 MPa verpresst (vorliegend z.B. bei 800 MPa), sodass ein Pressling entsteht. Anschließend erfolgt ein Vorsintern des Presslings bei 500 bis 1000 °C (vorliegend z.B. bei 900°C) für 20 min (Zeit bei Maximaltemperatur) unter Wasserstoffatmosphäre in einem Bandofen zum Zweck der Entwachsung des Presslings. Nach dem Vorsintern erfolgt ein Hochtemperatur-Sintern des Bauteils bei 1.100 °C bis 1450°C (vorliegend z.B. bei 1.450 °C) für 1-7 h (Zeit bei Maximaltemperatur; vorliegend z.B. über 7 h) unter Wasserstoffatmosphäre mit dem Zweck einer weiteren Verdichtung und Legierungsbildung. Nachfolgend wird eine Oxidation des Bauteils bei 950°C für eine Zeitdauer von 10 bis 30 h (vorliegend z.B. über 20 h; h: Stunde) durchgeführt, um eine eventuell vorhandene Restporosität soweit zu verschließen, dass die Permeabilität ausreichend niedrig ist. Die Oberfläche des oxidierten Bauteils wird von der Oxidschicht durch einen allseitigen Sandstrahlprozess befreit.

Ausführungsbeispiel Zweifaches Pressen:

Die Vorteile und weitere Hinweise zu dem zweistufigen Pressvorgang sind z.B. in der US 8,173,063 B2 beschrieben. Die Chrompulvergewinnung sowie die Herstellung des Pulveransatzes erfolgt wie oberhalb erläutert. Die Herstellung des Presslings einschließlich des Schrittes des Pressens und der Schritt des Vorsinterns erfolgen wie bei dem Ausführungsbeispiel Einfaches Pressen oberhalb. Nach dem Vorsintern wird ein Kalibrierpressen des vorgesinterten Bauteils bei einem spezifischen Pressdruck von 500 bis 1000 MPa (vorliegend z.B. bei 800 MPa) durchgeführt. Anschließend werden ein Hochtemperatur-Sintern, eine Oxidation sowie eine Bearbeitung mit einem Sandstrahlprozess entsprechend wie bei dem Ausführungsbeispiel Einfaches Pressen durchgeführt.

In Bezug auf den Pressdruck bei dem Schritt des Pressens ist zu ergänzen, dass bei Verwendung eines Pulvers mit einer großen Oberfläche nach BET (insbesondere $\geq$ 0,05 m$^2$/g) und besonders bei Verwendung eines schwammartigen Pulvers, wie es z.B. gemäß dem Verfahren nach Anspruch 19 herstellbar ist (vgl. insbesondere das oberhalb erläuterte Verfahren zur Chrompulvergewinnung), eine deutlich bessere Pressbarkeit gegeben ist und dementsprechend niedrigere Pressdrücke ausreichend sind. Dies ist vorteilhaft im Hinblick auf die Herstellungskosten sowie im Hinblick auf eine möglichst geringe Abnutzung der Presswerkzeuge. Während bei einer maximalen Dicke im Bereich des/der Strömungsfelder von 2,5 mm und bei herkömmlichem, aluminothermisch hergestellten Chrompulver typischerweise ein Pressdruck von > 900 MPa (MPa: Megapascal) angewendet wird, ist bei Verwendung des vorteilhaften Pulvers sowohl für eine maximale Dicke von 2,5 mm als auch von 2,2 mm ein Pressdruck von 600 MPa ausreichend. Bei einer maximalen Dicke im Bereich des/der Strömungsfelder von 2,0 mm und von 1,8 mm war bei Verwendung des vorteilhaften Pulvers sogar ein Pressdruck von nur 500 MPa ausreichend.

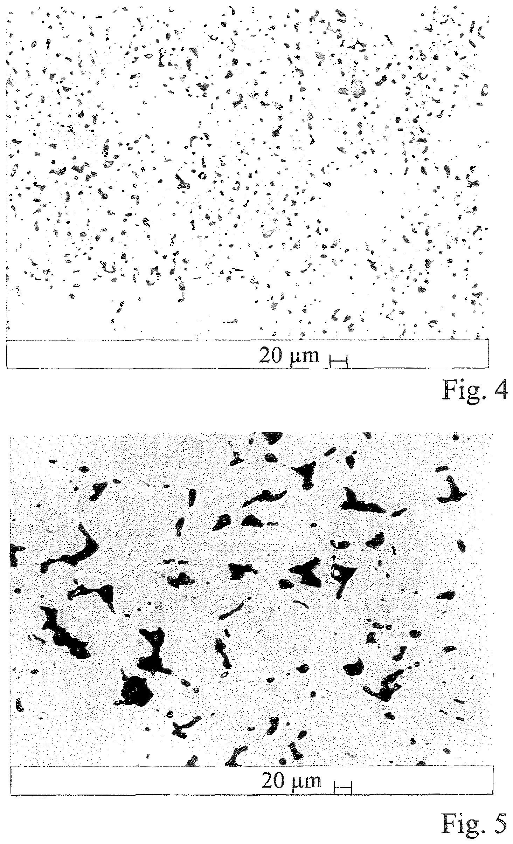

Weiterhin weist das Formteil bei Verwendung eines Pulvers mit einer großen Oberfläche nach BET (insbesondere $\geq$ 0,05 m$^2$/g) und besonders bei Verwendung eines schwammartigen Pulvers, wie es z.B. gemäß dem Verfahren nach Anspruch 19 herstellbar ist (vgl. insbesondere das oberhalb erläuterte Verfahren zur Chrompulvergewinnung) eine besonders vorteilhafte Mikrostruktur auf, wie nachfolgend unter Bezugnahme auf die Fig. 4 und 5 erläutert wird. In Fig. 4 ist dabei eine rasterelektronenmikroskopische Aufnahme eines Querschnitt-Schliffes eines Interkonnektors, der solch eine vorteilhafte Mikrostruktur aufweist, dargestellt. Im Vergleich dazu ist in Fig. 5 eine entsprechende Aufnahme eines herkömmlich hergestellten Interkonnektors (unter Verwendung von aluminothermisch hergestelltem Chrompulver) im gleichen Maßstab gezeigt. Die leeren oder teilweise mit Metalloxid(en) gefüllten Poren sowie die Oxideinschlüsse sind in diesen Aufnahmen deutlich dunkler als die umgebende, metallische Matrix. Wie anhand der Figuren 4 und 5 ersichtlich ist, zeichnet sich die vorteilhafte Mikrostruktur durch ein fein verteiltes Porenmuster aus. Speziell der Anteil großer (leerer oder teilweise mit Metalloxid(en) gefüllter) Poren und Oxideinschlüsse ist sehr klein. Insbesondere ist - bezogen auf eine bestimmte Mindestgröße der jeweils betrachteten leeren oder teilweise mit Metalloxid(en) gefüllten Poren und Oxideinschlüsse - der Flächenanteil derselben relativ zu der Gesamtfläche an leeren oder teilweise mit Metalloxid(en) gefüllten Poren und Oxideinschlüssen bei der vorteilhaften Mikrostruktur deutlich kleiner als bei herkömmlich hergestellten Interkonnektoren, wie die nachfolgende Tabelle zeigt (ausgewertet mittels der nachfolgend beschriebenen quantitativen Bildanalyse):

| Mindestgröße | $\geq$ 100 $\mu m^2$ | $\geq$ 70 $\mu m^2$ | $\geq$ 50 $\mu m^2$ |
|---|---|---|---|
| herkömmlicher Interkonnektor | 88% | 92% | 94% |
| Interkonnektor hergestellt mit neuem Pulver | 5% | 14% | 24% |

Beschreibung der quantitativen Bildanalyse zur Bestimmung_der Mikrostruktur

Für die quantitative Bildanalyse wurden die Formteile mit einer Diamantdrahtsäge senkrecht zu deren Hauptfläche in Segmente mit ca. 20 mm Kantenlänge geschnitten. Die Schnittfläche wurde durch das/die Strömungsfeld(er) gelegt. Die Zuschnitte wurden mit Wasser gereinigt und nachfolgend getrocknet. Die trockenen Zuschnitte wurden in Epoxidharz eingebettet. Nach einer Aushärtezeit von mind. 8 Stunden wurden die Schnittkante der Proben metallografisch präpariert, d.h. es kann später eine Untersuchung über die Bauteilstärke hinweg erfolgen. Die Präparation umfasst die Schritte:

- Schleifen bei 150-240 N mit festgebundenem SiC- Papier mit den Körnungen 240, 320, 400, 800, 1000, 1200 und 2400 grit.
- Feinschleifen mit 9 $\mu$m $Al_2O_3$ Läpppapier.
- Polieren mit Diamant-Suspensionen zunächst mit 3 $\mu$m Körnung, dann mit 1 $\mu$m Körnung.
- Finales Polieren mit einer Diamant-Suspension der Körnung 0,04 $\mu$m.
- Reinigung der Proben im Ultraschallbad.
- Trocknung der Proben.

Anschließend wurden je Probe fünf Bilder von unterschiedlichen, repräsentativen Bereichen der Schlifffläche angefertigt, wobei die Bereiche jeweils innerhalb des Kernbereichs gewählt wurden. Dies geschah mittels Rasterelektronenmikroskopie ("Ultra Plus 55" der Firma Zeiss) unter Verwendung eines 4-Quadranten-Ring-Detektors zur Detektion rückgestreuter Elektronen (BSE: back-scattered-electrons; deutsch: rückgestreute Elektronen). Die Anregungsspannung betrug 20 kV, der Kippwinkel 0°. Die Aufnahmen wurden scharf gestellt, die Auflösung sollte zumindest 1024x768 Pixel für eine korrekte Bildanalyse betragen. Der Kontrast wurde derart gewählt, dass sich sowohl die Poren als auch eventuell vorhandene teilweise Metalloxid-Füllungen der Poren sowie Oxideinschlüsse deutlich von der metallischen Matrix abheben und - wie oberhalb erläutert - zusammengefasst ausgewertet werden können. Die Vergrößerung für die Aufnahmen wurde derart gewählt, dass jedes Bild zumindest 100 Poren/Oxideinschlüsse enthält. Daraus ergaben sich im vorliegenden Fall Bildflächen von 0,04 bis 0,25 mm$^2$.

Die quantitative Bildanalyse wurde mit der Software "QWin" von Leica durchgeführt. Es wurde das Modul "QXCount" genutzt. Jede Bildanalyse folgte den Schritten:

- Einstellung eines Graustufenschwellwertes in der Art, dass offenes und gegebenenfalls teilweise mit Metalloxid(en) gefülltes Porenvolumen in den Poren sowie auch Oxideinschlüsse (d.h. ohne offenes Porenvolumen) gemeinsam als "Pore/Oxideinschluss" erkannt wurden.
- Festlegung des Messrahmens, in diesem Fall der gesamten Bildfläche.
- Messoptionen: Klassifizierung nach Äquivalentdurchmesser.
- Detektionseinstellung: dunkle Objekte, Löcher füllen, Randteilchen entfernen, open reconstruct.

Es sollen weder bei der Aufnahme noch bei der Analyse der Aufnahmen Filterfunktionen verwendet werden. Da die "Poren/Oxideinschlüsse" (wie oben definiert) in einem Rückstreuelektronenbild dunkler erscheinen als die metallische Matrix müssen bei der Detektionseinstellung die "dunklen Objekte" als "Poren/Oxideinschlüsse" (wie oben definiert) definiert werden. Es kann vorkommen, beispielsweise aufgrund einer teilweisen Verfüllung des Porenvolumens mit Metalloxid(en), dass diese Kombination aus Porenvolumen und Metalloxidfüllung nicht als ein Objekt und damit als eine "Fläche" (für die oberhalb erläuterte Auswertung der Flächengrößen der Poren/Oxideinschlüsse) erkannt wird. Es ist die Option "Löcher füllen" zu verwenden, um diese Kombination und damit ihre Fläche als ein zusammengehöriges Objekt zu erfassen. Durch die Option "Randteilchen entfernen werden unvollständige "Poren/Oxideinschlüsse" (wie oben definiert) im Randbereich der Bildfläche nicht in die Auswertung einbezogen.

Nachdem die jeweils 5 Bilder einzeln analysiert worden sind, erfolgte eine statistische Auswertung über die Daten aller 5 Bilder hinweg. Für diese Auswertung wurden folgende Kenngrößen herangezogen:

- Flächenanteil der Poren (%)
- Dichte (1/mm$^2$) der Poren/Oxideinschlüsse (wie oben definiert)
- Äquivalenter Durchmesser ($\mu$m) der einzelnen Poren/Oxideinschlüsse
- Fläche ($\mu$m$^2$) der einzelnen Poren/Oxideinschlüsse

**Patentansprüche**

**1.** Ein einen Interkonnektor (2) oder eine Endplatte (36) für eine elektrochemische Zelle bildendes, pulvermetallurgisches Formteil, das einen Chromgehalt von mindestens 80 Gew.%, eine plattenförmige Grundform und (ein) an einer oder an beiden Hauptflächen (4, 26) des Formkörpers ausgebildete(s) Strömungsfeld(er) (6, 28) aufweist, wobei die jeweilige Hauptfläche (4, 26) in dem Bereich des/der Strömungsfeldes/Strömungsfelder (6, 28) jeweils eine in dem Formkörper ausgebildete Strukturierung (5, 30) mit einer Mehrzahl von noppen- oder stegförmigen Erhebungen (20, 32) und dazwischen liegenden Vertiefungen (22, 34) aufweist,
**dadurch gekennzeichnet,**
**dass** ein Verhältnis aus dem maximalen, entlang der Hauptfläche (4, 26) gemessenem Durchmesser $D_{max}$ des Formteils relativ zu einer minimalen Dicke $d_{min}$ eines sich entlang des/der Strömungsfeldes/Strömungsfelder (6, 28) erstreckenden und von der/den Strukturierung(en) (5, 30) nicht betroffenen Kernbereiches des Formteils in dem Bereich $140 \leq D_{max}/d_{min} \leq 350$ liegt.

2. Formteil gemäß Anspruch 1, **dadurch gekennzeichnet, dass** jeweils an beiden Hauptflächen (4, 26) des Formteils genau ein Strömungsfeld (6, 28), gegebenenfalls mit einem das Strömungsfeld (6, 28) vollständig oder teilweise umgebenden Randbereich (12), ausgebildet ist.

3. Formteil gemäß Anspruch 1 oder 2, **dadurch gekennzeichnet, dass** an beiden Hauptflächen (4, 26) des Formteils jeweils (ein) Strömungsfeld(er) (6, 28) ausgebildet ist/sind und dass die Strukturierungen (5, 30) von einander gegenüberliegenden Strömungsfeldern (6, 28) im Wesentlichen parallel zueinander verlaufende Haupterstreckungsrichtungen entlang der Hauptflächen (4, 26) aufweisen.

4. Formteil gemäß einem der vorangehenden Ansprüche, **dadurch gekennzeichnet, dass** das Formteil vier umlaufende Seitenkanten (8, 10, 16, 18) aufweist, von denen jeweils zwei einander gegenüberliegende Seitenkanten (8, 10, 16, 18) parallel zueinander verlaufen, wobei das Verhältnis aus Seitenlänge $L_{max}$ der längsten Seitenkante relativ zu der minimalen Dicke des Kernbereiches $d_{min}$ des Formteils $\geq 110$ ist.

5. Formteil gemäß einem der vorangehenden Ansprüche, **dadurch gekennzeichnet, dass** das Verhältnis aus der Größe der Hauptfläche (4, 26) in mm² (Quadratmillimeter) relativ zu der minimalen Dicke $d_{min}$ des Kernbereiches des Formteils in mm (Millimeter) $\geq 1{,}3 \times 10^4$ ist.

6. Formteil gemäß einem der vorangehenden Ansprüche, **dadurch gekennzeichnet, dass** das Verhältnis aus Gesamtgewicht des Formteils in g (Gramm) relativ zu der Größe der Hauptfläche (4, 26) in cm² (Quadratzentimeter) $\leq 1{,}1$ ist.

7. Formteil gemäß einem der vorangehenden Ansprüche, **dadurch gekennzeichnet,. dass** die maximale Dicke $d_{max}$ des Formteils in dem Bereich des/der Strömungsfeldes/Strömungsfelder (6, 28) $\leq 2{,}3$ mm (Millimeter) ist.

8. Formteil gemäß einem der vorangehenden Ansprüche, **dadurch gekennzeichnet, dass** die minimale Dicke $d_{min}$ des Kernbereichs des Formteils $\leq 1{,}1$ mm (Millimeter) ist.

9. Formteil gemäß einem der vorangehenden Ansprüche, **dadurch gekennzeichnet, dass** der maximale, entlang der Hauptfläche (4, 26) gemessene Durchmesser $D_{max}$ des Formteils $\geq 200$ mm ist.

10. Formteil gemäß einem der vorangehenden Ansprüche, **dadurch gekennzeichnet, dass** in dem Kernbereich der Flächenanteil von leeren oder teilweise mit Metalloxid(en) gefüllten Poren sowie von Oxideinschlüssen, die jeweils eine Fläche von $\geq 100$ $\mu m^2$ (Quadratmikrometer) aufweisen, relativ zu der Gesamtfläche an leeren oder teilweise mit Metalloxid(en) gefüllten Poren und Oxideinschlüssen $\leq 60$ % ist, ausgewertet mittels quantitativer Bildanalyse an einer rasterelektronenmikroskopischen Aufnahme einer Messfläche, die in eine entlang der Dickenrichtung verlaufende Schnittfläche durch das Formteil in den Kernbereich gelegt wird.

11. Formteil gemäß einem der vorangehenden Ansprüche, **dadurch gekennzeichnet, dass** in dem Kernbereich der Flächenanteil von leeren oder teilweise mit Metalloxid(en) gefüllten Poren sowie von Oxideinschlüssen, die jeweils eine Fläche von $\geq 70$ $\mu m^2$ (Quadratmikrometer) aufweisen, relativ zu der Gesamtfläche an leeren oder teilweise mit Metalloxid(en) gefüllten Poren und Oxideinschlüssen $\leq 70$ % ist, ausgewertet mittels quantitativer Bildanalyse an einer rasterelektronenmikroskopischen Aufnahme einer Messfläche, die in eine entlang der Dickenrichtung verlaufende Schnittfläche durch das Formteil in den Kernbereich gelegt wird.

**12.** Formteil gemäß einem der vorangehenden Ansprüche, **dadurch gekennzeichnet, dass** in dem Kernbereich der Flächenanteil von leeren oder teilweise mit Metalloxid(en) gefüllten Poren sowie von Oxideinschlüssen, die jeweils eine Fläche von $\geq 50\ \mu m^2$ (Quadratmikrometer) aufweisen, relativ zu der Gesamtfläche an leeren oder teilweise mit Metalloxid(en) gefüllten Poren und Oxideinschlüssen $\leq 80\ \%$ ist, ausgewertet mittels quantitativer Bildanalyse an einer rasterelektronenmikroskopischen Aufnahme einer Messfläche, die in eine entlang der Dickenrichtung verlaufende Schnittfläche durch das Formteil in den Kernbereich gelegt wird.

**13.** Formteil gemäß einem der vorangehenden Ansprüche, **dadurch gekennzeichnet, dass** in dem Kernbereich der Flächenanteil P von leeren oder teilweise mit Metalloxid(en) gefüllten Poren sowie von Oxideinschlüssen, soweit sie vollständig innerhalb der betrachteten Messfläche liegen, relativ zu der Gesamtfläche dieser Messfläche in einem Bereich von $80\% \leq (1-P) \leq 95\%$ liegt, ausgewertet mittels quantitativer Bildanalyse an einer rasterelektronenmikroskopischen Aufnahme einer Messfläche, die in eine entlang der Dickenrichtung verlaufende Schnittfläche durch das Formteil in den Kernbereich gelegt wird.

**14.** Formteil gemäß einem der vorangehenden Ansprüche, **dadurch gekennzeichnet, dass** in dem Kernbereich eine gleichmäßige Verteilung der leeren oder teilweise mit Metalloxid(en) gefüllten Poren und der Oxideinschlüsse vorliegt und dass in dem Kernbereich der mittlere Abstand $\lambda$ der leeren oder teilweise mit Metalloxid(en) gefüllten Poren und der Oxideinschlüsse $\leq 9\ \mu m$ ist, wobei der mittlere Abstand $\lambda$ nach der Formel

$$\lambda = \left(\frac{\frac{4}{3}\pi a^3}{P}\right)^{\frac{1}{3}}$$

berechnet wird,

wobei 2a dem mittleren, äquivalenten Poren- bzw. Oxideinschlussdurchmesser entspricht, wobei P der Flächenanteil von leeren oder teilweise mit Metalloxid(en) gefüllten Poren sowie von Oxideinschlüssen, soweit sie vollständig innerhalb einer Messfläche liegen, ist, und wobei die Größen 2a und P mittels quantitativer Bildanalyse an einer rasterelektronenmikroskopischen Aufnahme einer Messfläche ausgewertet werden, die in eine entlang der Dickenrichtung verlaufende Schnittfläche durch das Formteil in den Kernbereich gelegt wird.

**15.** Verfahren zum Herstellen eines einen Interkonnektor (2) oder eine Endplatte (36) für eine elektrochemische Zelle bildendes, pulvermetallurgisches Formteil, das einen Chromgehalt von mindestens 80 Gew.%, eine plattenförmige Grundform und (ein) an einer oder an beiden Hauptflächen (4, 26) des Formkörpers ausgebildete(s) Strömungsfeld(er) (6, 28) aufweist, wobei die jeweilige Hauptfläche (4, 26) in dem Bereich des/der Strömungsfeldes/Strömungsfelder (6, 28) jeweils eine in dem Formkörper ausgebildete Strukturierung (5, 30) mit einer Mehrzahl von noppen- oder stegförmigen Erhebungen (20, 32) und dazwischen liegenden Vertiefungen (22, 34) aufweist und wobei ein Verhältnis aus dem maximalen, entlang der Hauptfläche (4, 26) gemessenem Durchmesser $D_{max}$ des Formteils relativ zu einer minimalen Dicke $d_{min}$ eines sich entlang des/der Strömungsfeldes/Strömungsfelder (6, 28) erstreckenden und von der/den Strukturierung(en) (5, 30) nicht betroffenen Kernbereiches des Formteils in dem Bereich $140 \leq D_{max}/d_{min} \leq 350$ liegt, aufweisend die Schritte:

A) Bereitstellen eines Pulveransatzes, der bezogen auf den Gesamtmetallgehalt einen Chromanteil von $\geq 80$ Gew.% enthält und bei dem zumindest ein Anteil des Chrom enthaltenden Pulvers eine Oberfläche nach BET von $\geq 0,05\ m^2/g$ aufweist;
B) Pressen des Pulveransatzes zu einem Pressling;
C) Sintern des Presslings bei 1.100 bis 1.500 °C.

**16.** Verfahren gemäß Anspruch 15, **dadurch gekennzeichnet, dass** zwischen dem Schritt des Pressens (Schritt B) und dem Schritt des Sinterns (Schritt C) ein Vorsintern des Presslings bei 500 bis 1.000 °C erfolgt.

**17.** Verfahren gemäß Anspruch 15 oder 16, **dadurch gekennzeichnet, dass** das nach dem Schritt des Sinterns (Schritt C) erhaltene Formteil unter Anwesenheit einer Sauerstoffquelle oxidiert wird und anschließend die Oxidschicht von zumindest einem Teil der Oberfläche des Formteils entfernt wird.

**18.** Verfahren gemäß einem der Ansprüche 15 bis 17, **dadurch gekennzeichnet, dass** der Schritt des Pressens (Schritt B) den einzigen, während der Herstellung des Formteils durchgeführten Pressvorgang bildet.

**19.** Verfahren gemäß einem der Ansprüche 15 bis 18, **dadurch gekennzeichnet, dass** im Rahmen des Schrittes des Bereitstellens (Schritt A) ein Chrom enthaltendes Pulver mit einer Oberfläche nach BET von $\geq 0{,}05$ $m^2/g$ und einem Chromgehalt von $\geq 90$ Gew.% durch Reduktion zumindest einer Verbindung der Gruppe bestehend aus Chromoxid und Chromhydroxid, optional mit einer beigemischten festen Kohlenstoffquelle, unter zumindest zeitweiser Einwirkung von Wasserstoff und Kohlenwasserstoff hergestellt wird und zumindest anteilig dem Pulveransatz zugegeben wird.

**Claims**

**1.** A powder metallurgical moulding forming an interconnector (2) or an end plate (36) for an electrochemical cell, which moulding has a chromium content of at least 80% by weight, a plate-like basic shape and (a) flow field(s) (6, 28) formed on one or both of the main faces (4, 26) of the moulding, wherein the main face (4, 26) has in the region of the flow field(s) (6, 28) a structuring (5, 30) formed in the moulding with a plurality of knob- or ridge-like elevations (20, 32) and intermediate depressions (22, 34), **characterized in that**
a ratio of the maximum diameter $D_{max}$ of the moulding, measured along the main face (4, 26), to a minimum thickness $d_{min}$ of a core region of the moulding which extends along the flow field(s) (6, 28) and is not affected by the structuring(s) (5, 30) is in the range $140 \leq D_{max}/d_{min} \leq 350$.

**2.** Moulding according to Claim 1, **characterized in that** precisely one flow field (6, 28) is formed on both of the main faces (4, 26) of the moulding, optionally with an edge region (12) which surrounds the flow field (6, 28) completely or partially.

**3.** Moulding according to Claim 1 or 2, **characterized in that** (a) flow field(s) (6, 28) is/are formed on both of the main faces (4, 26) of the moulding, and **in that** the structurings (5, 30) of mutually opposite flow fields (6, 28) have main extension directions along the main faces (4, 26) which run substantially parallel to one another.

**4.** Moulding according to one of the preceding claims, **characterized in that** the moulding has four circumferential side edges (8, 10, 16, 18), of which in each case two mutually opposite side edges (8, 10, 16, 18) run parallel to one another, wherein the ratio of the side length $L_{max}$ of the longest side edge to the minimum thickness of the core region $d_{min}$ of the moulding is $\geq 110$.

**5.** Moulding according to one of the preceding claims, **characterized in that** the ratio of the size of the main face (4, 26) in $mm^2$ (square millimetres) to the minimum thickness $d_{min}$ of the core region of the moulding in mm (millimetres) is $\geq 1.3 \times 10^4$.

**6.** Moulding according to one of the preceding claims, **characterized in that** the ratio of the total weight of the moulding in g (grams) to the size of the main face (4, 26) in $cm^2$ (square centimetres) is $\leq 1.1$.

**7.** Moulding according to one of the preceding claims, **characterized in that** the maximum thickness $d_{max}$ of the moulding in the region of the flow field/fields (6, 28) is $\leq 2.3$ mm (millimetres).

**8.** Moulding according to one of the preceding claims, **characterized in that** the minimum thickness $d_{min}$ of the core region of the moulding is $\leq 1.1$ mm (millimetres).

**9.** Moulding according to one of the preceding claims, **characterized in that** the maximum diameter $D_{max}$ of the moulding, measured along the main face (4, 26), is $\geq 200$ mm.

**10.** Moulding according to one of the preceding claims, **characterized in that**, in the core region, the proportion by surface area of pores that are empty or partly filled with metal oxide(s) and of oxide inclusions which have a surface area of $\geq 100$ $\mu m^2$ (square micrometres), relative to the total surface area of pores that are empty or partly filled with metal oxide(s) and of oxide inclusions, is $\leq 60\%$, evaluated by means of quantitative image analysis on a scanning electron microscope image of a measurement area which is located in a cut surface, running along the thickness direction, through the moulding into the core region.

**11.** Moulding according to one of the preceding claims, **characterized in that**, in the core region, the proportion by surface area of pores that are empty or partly filled with metal oxide(s) and of oxide inclusions which have a surface area of $\geq 70\ \mu m^2$ (square micrometres), relative to the total surface area of pores that are empty or partly filled with metal oxide(s) and of oxide inclusions, is $\leq 70\%$, evaluated by means of quantitative image analysis on a scanning electron microscope image of a measurement area which is located in a cut surface, running along the thickness direction, through the moulding into the core region.

**12.** Moulding according to one of the preceding claims, **characterized in that**, in the core region, the proportion by surface area of pores that are empty or partly filled with metal oxide(s) and of oxide inclusions which have a surface area of $\geq 50\ \mu m^2$ (square micrometres), relative to the total area of pores that are empty or partly filled with metal oxide(s) and of oxide inclusions, is $\leq 80\%$, evaluated by means of quantitative image analysis on a scanning electron microscope image of a measurement area which is located in a cut surface, running along the thickness direction, through the moulding into the core region.

**13.** Moulding according to one of the preceding claims, **characterized in that**, in the core region, the proportion by surface area P of pores that are empty or partly filled with metal oxide(s) and of oxide inclusions, provided they lie wholly within the measurement area under consideration, relative to the total surface area of the measurement area, is in a range $80\% \leq (1\text{-}P) \leq 95\%$, evaluated by means of quantitative image analysis on a scanning electron microscope image of a measurement area which is located in a cut surface, running along the thickness direction, through the moulding into the core region.

**14.** Moulding according to one of the preceding claims, **characterized in that**, in the core region, there is uniform distribution of pores that are empty or partly filled with metal oxide(s) and of oxide inclusions, and **in that**, in the core region, the mean spacing $\lambda$ of the pores that are empty or partly filled with metal oxide(s) and of the oxide inclusions is $\leq 9\ \mu m$, wherein the mean spacing $\lambda$ is calculated according to the formula

$$\lambda = \left( \frac{\frac{4}{3}\pi a^3}{P} \right)^{\frac{1}{3}},$$

wherein 2a corresponds to the mean equivalent pore or oxide inclusion diameter, wherein P is the proportion by surface area of pores that are empty or partly filled with metal oxide(s) and of oxide inclusions, provided they lie wholly within a measurement area, and

wherein the values 2a and P are evaluated by means of quantitative image analysis on a scanning electron microscope image of a measurement area which is located in a cut surface, running along the thickness direction, through the moulding into the core region.

**15.** Process for the production of a powder metallurgical moulding forming an interconnector (2) or an end plate (36) for an electrochemical cell, which moulding has a chromium content of at least 80% by weight, a plate-like basic shape and (a) flow field(s) (6, 28) formed on one or both of the main faces (4, 26) of the moulding, wherein the main face (4, 26) has in the region of the flow field(s) (6, 28) a structuring (5, 30) formed in the moulding with a plurality of knob- or ridge-like elevations (20, 32) and intermediate depressions (22, 34), **characterized in that** a ratio of the maximum diameter $D_{max}$ of the moulding, measured along the main face (4, 26), to a minimum thickness $d_{min}$ of a core region of the moulding which extends along the flow field(s) (6, 28) and is not affected by the structuring(s) (5, 30) is in the range $140 \leq D_{max}/d_{min} \leq 350$, comprising the steps:

A) providing a powder batch which, based on the total metal content, has a chromium content of $\geq 80\%$ by weight and in which at least a portion of the chromium-containing powder has a BET surface area of $\geq 0.05\ m^2/g$,
B) pressing the powder batch to form a compact,
C) sintering the compact at from 1100 to 1500°C.

**16.** Process according to Claim 15, **characterized in that** presintering of the compact at from 500 to 1000°C is carried out between the pressing step (step B) and the sintering step (step C).

**17.** Process according to Claim 15 or 16, **characterized in that** the moulding obtained after the sintering step (step C) is oxidized in the presence of an oxygen source, and then the oxide layer is removed from at least part of the surface of the moulding.

**18.** Process according to one of Claims 15 to 17, **characterized in that** the pressing step (step B) forms the only pressing operation carried out during the production of the moulding.

**19.** Process according to one of Claims 15 to 18, **characterized in that**, within the context of the providing step (step A), a chromium-containing powder having a BET surface area of $\geq 0.05$ m$^2$/g and a chromium content of $\geq 90\%$ by weight is produced by reduction of at least one compound from the group consisting of chromium oxide and chromium hydroxide, optionally with an added solid carbon source, under the at least temporary exposure of hydrogen and hydrocarbon and is added at least proportionately to the powder mixture.

**Revendications**

**1.** Pièce moulée de la métallurgie des poudres formant un interconnecteur (2) ou une plaque d'extrémité (36) pour une cellule électrochimique, laquelle présente une teneur en chrome d'au moins 80 % en poids, une forme de base en plaque et un/des champs d'écoulement (6, 28) réalisé/réalisés au niveau d'une ou au niveau de deux faces principales (4, 26) du corps moulé, dans laquelle la face principale (4, 26) respective présente, dans la zone du/des champs d'écoulement (6, 28), respectivement une structuration (5, 30) réalisée dans le corps moulé avec une multitude de parties surélevées (20, 32) en forme de boutons ou de nervures et de renfoncements (22, 34) intercalés, **caractérisée en ce qu'**un rapport entre le diamètre maximal $D_{max}$ de la pièce moulée mesuré le long de la face principale (4, 26) et une épaisseur minimale $d_{min}$ d'une zone centrale de la pièce moulée s'étendant le long du/des champs d'écoulement (6, 28) et non concernée par la/les structuration (s) (5, 30) se situe dans la plage $140 \leq D_{max}/d_{min} \leq 350$.

**2.** Pièce moulée selon la revendication 1, **caractérisée en ce que** précisément un champ d'écoulement (6, 28) comprenant éventuellement une zone de bord (12) entourant en totalité ou en partie le champ d'écoulement (6, 28) est réalisé respectivement au niveau des deux faces principales (4, 26) de la pièce moulée.

**3.** Pièce moulée selon la revendication 1 ou 2, **caractérisée en ce que** respectivement un/des champs d'écoulement (6, 28) est/sont réalisés au niveau des deux faces principales (4, 26) de la pièce moulée, et que les structurations (5, 30) de champs d'écoulement (6, 28) se faisant face les uns les autres présentent des directions d'extension principales le long des faces principales (4, 26) s'étendant sensiblement de manière parallèle les unes aux autres.

**4.** Pièce moulée selon l'une quelconque des revendications précédentes, **caractérisée en ce que** la pièce moulée présente quatre arêtes latérales (8, 10, 16, 18) périphériques, parmi lesquelles respectivement deux arêtes latérales (8, 10, 16, 18) se faisant face l'une l'autre s'étendent de manière parallèle l'une par rapport à l'autre, dans laquelle le rapport entre la longueur latérale $L_{max}$ de l'arête latérale la plus longue et l'épaisseur minimale $d_{min}$ de la zone centrale de la pièce moulée est $\geq 110$.

**5.** Pièce moulée selon l'une quelconque des revendications précédentes, **caractérisée en ce que** le rapport entre la taille de la face principale (4, 26) exprimée en mm$^2$ (millimètres carrés) et l'épaisseur minimale $d_{min}$ de la zone centrale de la pièce moulée exprimée en mm (millimètre) est $\geq 1,3$ x $10^4$.

**6.** Pièce moulée selon l'une quelconque des revendications précédentes, **caractérisée en ce que** le rapport entre le poids total de la pièce moulée en g (grammes) et la taille de la face principale (4, 26) en cm$^2$ (centimètres carrés) est $\leq 1,1$.

**7.** Pièce moulée selon l'une quelconque des revendications précédentes, **caractérisée en ce que** l'épaisseur maximale $d_{max}$ de la pièce moulée se situe dans la plage du/des champs d'écoulement (6, 28) $\leq 2,3$ mm (millimètres).

**8.** Pièce moulée selon l'une quelconque des revendications précédentes, **caractérisée en ce que** l'épaisseur minimale $d_{min}$ de la zone centrale de la pièce moulée est $\leq 1,1$ mm (millimètre).

**9.** Pièce moulée selon l'une quelconque des revendications précédentes, **caractérisée en ce que** le diamètre $D_{max}$ maximal de la pièce moulée mesuré le long de la face principale (4, 26) est $\geq 200$ mm.

**10.** Pièce moulée selon l'une quelconque des revendications précédentes, **caractérisée en ce que** la surface occupée par des pores vides ou remplis en partie d'oxyde(s) métallique(s) ainsi que par des inclusions d'oxyde, qui présentent respectivement une surface $\geq 100$ $\mu$m$^2$ (micromètres carrés) par rapport à la surface totale de pores vides ou remplis en partie d'oxydes métallique(s) et d'inclusions d'oxyde est, dans la zone centrale, $\leq 60\ \%$, laquelle est évaluée au moyen d'une analyse d'image quantitative sur une prise de vue, réalisée au microscope électronique à balayage, d'une face de mesure, qui est placée dans une face de coupe s'étendant le long du sens de l'épaisseur à travers la pièce moulée dans la zone centrale.

11. Pièce moulée selon l'une quelconque des revendications précédentes, **caractérisée en ce que** la surface occupée par des pores vides ou remplis en partie d'oxyde(s) métallique(s) ainsi par des inclusions d'oxyde, qui présentent respectivement une surface $\geq 70\ \mu m^2$ (micromètres carrés), par rapport à la surface totale de pores vides ou remplis en partie d'oxyde(s) métallique(s) et d'inclusions d'oxyde est, dans la zone centrale, $\leq 70\ \%$, laquelle est évaluée au moyen d'une analyse d'image quantitative sur une prise de vue, réalisée au microscope électronique par balayage, d'une face de mesure, qui est placée dans une face de coupe, s'étendant le long du sens de l'épaisseur, à travers la pièce moulée dans la zone centrale.

12. Pièce moulée selon l'une quelconque des revendications précédentes, **caractérisée en ce que** la surface occupée par des pores vides ou remplis en partie d'oxyde(s) métallique(s) ainsi que par des inclusions d'oxyde, qui présentent respectivement une surface $\geq 50\ \mu m^2$ (micromètres carrés), par rapport à la surface totale de pores vides ou remplis en partie d'oxyde(s) métallique(s) et d'inclusions d'oxyde est, dans la zone centrale, $\leq 80\ \%$, laquelle est évaluée au moyen d'une analyse d'image quantitative sur une prise de vue, réalisée au microscope électronique par balayage, d'une face de mesure, qui est placée dans une face de coupe, s'étendant le long du sens de l'épaisseur, à travers la pièce moulée dans la zone centrale.

13. Pièce moulée selon l'une quelconque des revendications précédentes, **caractérisée en ce que** la surface occupée P par des pores vides ou remplis en partie d'oxyde(s) métallique(s) ainsi que par des inclusions d'oxyde, dans la mesure où ils se trouvent en totalité à l'intérieur de la face de mesure considérée, par rapport à la surface totale de ladite face de mesure se situe, dans la zone centrale, dans une plage $80\ \% \leq (1\text{-}P) \leq 95\ \%$, laquelle est évaluée au moyen d'une analyse d'image quantitative sur une prise de vue, réalisée au microscope électronique par balayage, d'une face de mesure, qui est placée dans une face de coupe, s'étendant le long du sens de l'épaisseur, à travers la pièce moulée dans la zone centrale.

14. Pièce moulée selon l'une quelconque des revendications précédentes, **caractérisée en ce qu'**une répartition homogène des pores vides ou remplis en partie d'oxyde(s) métallique(s) et des inclusions d'oxyde est présente dans la zone centrale, et **en ce que** la distance moyenne $\lambda$ des pores vides ou en partie remplis d'oxyde(s) métallique(s) et des inclusions d'oxyde est, dans la zone centrale, $\leq 9\ \mu m$, dans laquelle la distance moyenne $\lambda$ est calculée selon la formule

$$\lambda = \left(\frac{\frac{4}{3}\pi a^3}{P}\right)^{\frac{1}{3}},$$

dans laquelle 2a correspond au diamètre moyen de pore ou d'inclusion d'oxyde équivalent, dans laquelle P est la surface occupée par des pores vides ou remplis en partie d'oxyde(s) métallique(s) ainsi que par des inclusions d'oxyde, dans la mesure où ils se trouvent en totalité à l'intérieur d'une face de mesure, et dans laquelle les grandeurs 2a et P sont évaluées au moyen d'une analyse d'image quantitative sur une prise de vue, réalisée au microscope électronique par balayage, d'une face de mesure, qui est placée dans une face de coupe, s'étendant le long du sens de l'épaisseur, à travers la pièce moulée dans la zone centrale.

15. Procédé servant à fabriquer une pièce moulée de la métallurgie des poudres formant un interconnecteur (2) ou une plaque d'extrémité (36) pour une cellule électrochimique, laquelle présente une teneur en chrome d'au moins 80 % en poids, une forme de base en plaque et un/des champs d'écoulement (6, 28) réalisé/réalisés au niveau d'une ou au niveau de deux faces principales (4, 26) du corps moulé, dans lequel la face principale (4, 26) respective présente, dans la zone du/des champs d'écoulement (6, 28), respectivement une structuration (5, 30) réalisée dans le corps moulé, avec une multitude de parties surélevées (20, 32) en forme de boutons ou de nervures et de renfoncements (22, 34) intercalés, et dans lequel un rapport entre le diamètre maximal $D_{max}$ de la pièce moulée mesuré le long de la face principale (4, 26) et une épaisseur minimale $d_{min}$ d'une zone centrale de la pièce moulée s'étendant le long du/des champs d'écoulement (6, 28) et non concernée par la/les structuration(s) (5, 30) se situe dans la plage $140 \leq D_{max}/d_{min} \leq 350$, présentant les étapes qui suivent consistant à :

A) fournir une ébauche de poudre, qui contient, par rapport à la teneur en métal totale, une proportion de chrome $\geq$ 80 % en poids et qui présente, pour la poudre contenant au moins une proportion de chrome, une surface selon la méthode BET $\geq$ 0,05 m$^2$/g ;
B) presser l'ébauche de poudre en un comprimé ;
C) fritter le comprimé à une température allant de 1 100 à 1 500 °C.

16. Procédé selon la revendication 15, **caractérisé en ce qu'**un préfrittage du comprimé est effectué à une température allant de 500 à 1 000 °C entre l'étape du pressage (étape B) et l'étape du frittage (étape C).

17. Procédé selon la revendication 15 ou 16, **caractérisé en ce que** la pièce moulée obtenue après l'étape du frittage (étape C) est oxydée en présence d'une source d'oxygène, et la couche d'oxyde est immédiatement après éliminée d'au moins une partie de la surface de la pièce moulée.

18. Procédé selon l'une quelconque des revendications 15 à 17, **caractérisé en ce que** l'étape du pressage (étape B) forme l'unique opération de pressage effectuée au cours de la fabrication de la pièce moulée.

19. Procédé selon l'une quelconque des revendications 15 à 18, **caractérisé en ce que** dans le cadre de l'étape de fourniture (étape A), une poudre contenant du chrome présentant une surface selon la méthode BET $\geq$ 0,05 m$^2$/g et une teneur en chrome $\geq$ 90 % en poids est fabriquée par réduction d'au moins un composé du groupe constitué de l'oxyde de chrome et de l'hydroxyde de chrome, en option avec une source de carbone solide mélangée, au moins sous l'action temporaire d'hydrogène et d'hydrocarbure et est mélangée au moins de manière proportionnelle à l'ébauche de poudre.

Fig. 1

Fig. 2

Fig. 3

20 µm ⊢

Fig. 4

20 µm ⊢

Fig. 5

**IN DER BESCHREIBUNG AUFGEFÜHRTE DOKUMENTE**

**In der Beschreibung aufgeführte Patentdokumente**

- US 5407758 A
- US 20100233576 A1
- US 8173063 B2